# EUROPEAN PATENT APPLICATION

(11) **EP 2 397 458 A1**
(43) Date of publication of application: **21.12.2011**
(21) Application number: 10006440.1
(22) Date of filing: 21.06.2010
(51) Int. Cl.: C07C 51/41, C07C 57/30, C07C 65/05, C07C 211/27, A61K 31/19, A61K 31/205

(54) **Organic salts and co-crystals of phenylbutyric acid**

(71) Applicant: Lunamed AG, 7000 Chur (CH)
(72) Inventor: Truog, Peter, 7000 Chur (CH); Hett, Robert, 4132 Muttenz (CH)
(74) Representative: Peters, Hajo

(57) **Abstract**

The present invention refers to joint mixtures of phenylbutyric acid or its ions and at least one organic molecule, like organic salts of phenylbutyrate, polymorphs, hydrates or solvates thereof and co-crystals of phenylbutyric acid and at least one organic molecule, polymorphs, hydrates or solvates thereof, as well as their use in cancer therapy and other pharmaceutical applications. Preferred organic compounds are benethamine, benzathine, benzylamine and gentisic acid.

## Description

### Field of the invention

The present invention refers to joint mixtures of phenylbutyric acid or its ions and at least one organic molecule, like organic salts of phenylbutyric acid, polymorphs, hydrates or solvates thereof and co-crystals of phenylbutyric acid with at least one organic molecule, polymorphs, hydrates or solvates thereof, as well as their use in cancer therapy and other pharmaceutical applications.

### Background of the invention

Phenylbutyric acid is a well known compound to which a number of medical uses has been ascribed in patent or scientific literature. In form of its sodium salt it is marketed as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

Over the years a number of salts of phenylbutyric acid has been described. Still, except for sodium phenyl butyrate none of them has - for various reasons - reached the market. On the other hand the marketed sodium phenylbutyrate can be - especially in the indication marketed for but also in other circumstances **-** quite disadvantageous. According to Summary of the Product Characteristics (ANNEX I published by the EMEA) under 4.4 (Special Warnings) it is stated:
*"Each AMMONAPS tablet contains 62 mg (2.7 mmol) of sodium, corresponding to 2.5 g (108 mmol) of sodium per 20 g of sodium phenylbutyrate, which is the maximum daily dose. AMMONAPS should therefore be used with caution in patients with congestive heart failure or severe renal insufficiency, and in clinical conditions where there is sodium retention with oedema. "*

In addition, it was further intended to identify a form of the active ingredient that would allow a favorable use in a slow-release formulation. Sodium phenylbutyrate is very well soluble in water. Even though this is in general a quite positive attribute, for this intention an API that is still water soluble but less so than sodium phenylbutyrate might have its advantageous, especially if easy to formulate due to its crystallinity.

Accordingly, it was the goal of this invention to identify alternative joint mixtures of phenylbutyric acid or its ions, especially like organic salts of phenylbutyric acid, polymorphs, hydrates or solvates thereof and co-crystals of phenylbutyric acid, polymorphs, hydrates or solvates thereof, to substitute the sodium in sodium phenylbutyrate, especially joint mixtures that are water soluble, though less so than sodium phenylbutyrate and/or joint mixtures that are crystalline.

It has now been found that joined mixture of phenylbutyric acid or its inorganic salts and at least one organic molecule, wherein the mixture is joined by intermolecular physically interacting forces, especially fitting salts or co-crystals are very advantageous.

Accordingly, the invention is drawn to a joined mixture of phenylbutyric acid or its inorganic salts and at least one organic molecule, wherein the mixture is joined by intermolecular physically interacting forces.

"Joined mixture" according to the invention is defined as a mixture of the phenylbutyric acid or its inorganic salts and at least one organic molecule mixture in which it is not only a physical mixture without molecular interaction but is joined by intermolecular physically interacting forces, like ion-ion-interaction, van der Waals-Force, a hydrogen bond, or a disulfide bond. "Joined mixtures" include a) a salt of phenylbutyrate with a positively charged ion of an organic molecule including a respective polymorph, hydrate or solvate of the salt; b) a salt of phenylbutyrate with a positively charged ion of an organic molecule; c) a polymorph of a salt of phenylbutyrate with a positively charged ion of an organic molecule; d) a hydrate of a salt of phenylbutyrate with a positively charged ion of an organic molecule; e) a solvate of a salt of phenylbutyrate with a positively charged ion of an organic molecule; f) a co-crystal of phenylbutyric acid and at least one organic molecule as co-crystal former, including a respective polymorph, hydrate or solvate of the co-crystal; g) a co-crystal of phenylbutyric acid and at least one organic molecule as co-crystal former; h) a polymorph of a co-crystal of phenylbutyric acid and at least one organic molecule as co-crystal former; i) a hydrate of a co-crystal of phenylbutyric acid and at least one organic molecule as co-crystal former; or j) a solvate of a co-crystal of phenylbutyric acid and at least one organic molecule as co-crystal former.

"Intermolecular physically interacting forces" is defined in this application as forces that physically interact in the different parts of the joint mixture for example by ion-ion-interaction, van der Waals-Force, a hydrogen bond, or a disulfide bond.

Any, all or any mixture of the following provisos may apply:
● joined mixtures of phenylbutyric acid with ammonium are excluded and/or
● joined mixtures of phenylbutyric acid with 1-carboxy-N,N,N-trimethyl-methanaminium are excluded; and/or
● salts of phenylbutyric acid with arginine are excluded; and/or
● salts of phenylbutyric acid with lysine are ecluded; and/or
● salts of phenylbutyric acid with choline are excluded; and/or
● salts of phenylbutyric acid with glycine are excluded; and/or
● salts of phenylbutyric acid with alanine are excluded; and/or
● salts of phenylbutyric acid with asparagine are excluded; and/or
● salts of phenylbutyric acid with glutamine are excluded; and/or
● salts of phenylbutyric acid with histidine are excluded; and/or
● co-crystals of phenylbutyric acid with sodium phenylbutyrate.

In a preferred embodiment of the invention the joined mixture according to the invention is a crystalline form of phenylbutyric acid or its ions and at least one organic molecule.

In another preferred embodiment of the joined mixture according to the invention, the joined mixture is selected from
● a salt of phenylbutyrate with a positively charged ion of an organic molecule, including a respective polymorph, hydrate or solvate of the salt;
● a salt of phenylbutyrate with a positively charged ion of an organic molecule;
● a polymorph of a salt of phenylbutyrate with a positively charged ion of an organic molecule;
● a hydrate of a salt of phenylbutyrate with a positively charged ion of an organic molecule;
● a solvate of a salt of phenylbutyrate with a positively charged ion of an organic molecule;
● a co-crystal of phenylbutyric acid and at least one organic molecule as co-crystal former including a respective polymorph, hydrate or solvate of the co-crystal;
● a co-crystal of phenylbutyric acid and at least one organic molecule as co-crystal former;
● a polymorph of a co-crystal of phenylbutyric acid and at least one organic molecule as co-crystal former;
● a hydrate of a co-crystal of phenylbutyric acid and at least one organic molecule as co-crystal former; or
● a solvate of a co-crystal of phenylbutyric acid and at least one organic molecule as co-crystal former.

"Polymorphs" in general are described in "Polymorphism in Pharmaceutical Solids", Harry G. Brittain (ed.) Drugs and the Pharmaceutical Sciences 192 (1999).

In a further preferred embodiment of the joined mixture according to the invention the organic molecule is selected from
● ammonia and amines,
● amino acids,
● carboxylic acids, or
● heterocylic compounds

Preferably, in a further preferred embodiment of the joined mixture according to the invention, the organic molecule is selected from
● ammonia;
● amines, like 2-(diethylamino)-ethanol, benethamine, benzathine, benzylamine, betaine (trimethylglycine), choline, deanol, diethanolamine, diethylamine (2,2'-iminobis(ethanol)), ethanolamine (2-aminoethanol), ethylenediamine, glucamines, like N-methyl-glucamine, hydrabramine, tromethamine, triethanolamine (2,2',2"-nitrolitris(ethanol)):
● carbocylic acids, like benzoic acid, citric acid, gentisic acid, glycolic acid, lactic acid, naphtoic acids, succinic acid, tartaric acid, or vanillic acid;
● D-aminoacids like D-alanine:
● L-amino acids like L-lysine or L-arginine; or
● heterocyclic compounds, like imidazoles, like 1 H-Imidazole; morpholines, like 4-(2-hydroxyethyl)-morpholine; piperazine; pyrrolidines, like 1-(2-hydroxyethyl)-pyrrolidine.

In a further preferred embodiment of the joined mixture according to the invention the organic molecule is selected from
● 2-(diethylamino)-ethanol,
● ammonia,
● benethamine,
● benzathine,
● benzoic acid,
● benzylamine,
● betaine (trimethylglycine),
● choline,
● citric acid,
● D-alanine,
● deanol,
● diethanolamine,
● diethylamine 2,2'-iminobis(ethanol)),
● ethanolamine (2-aminoethanol),
● ethylenediamine,
● gentisic acid,
● glucamines, like N-methyl-glucamine,
● glycolic acid,
● hydrabramine,
● imidazoles, like 1 H-Imidazole,
● lactic acid,
● L-amino acids like L-lysine or L-arginine,
● morpholines, like 4-(2-hydroxyethyl)-morpholine,
● naphtoic acids,
● piperazine,
● pyrrolidines, like 1-(2-hydroxyethyl)-pyrrolidine,
● succinic acid,
● tartaric acid,
● tromethamine,
● triethanolamine (2,2',2"-nitrolitris(ethanol)), or
● vanillic acid;
preferably is selected from
● 1-(2-hydroxyethyl)-pyrrolidine,
● 2-(diethylamino)-ethanol,
● 4-(2-hydroxyethyl)-morpholine,
● benethamine,
● benzathine,
● benzoic acid,
● benzylamine,
● betaine (trimethylglycine),
● choline,
● citric acid,
● deanol,
● diethylamine,
● ethanolamine,
● gentisic acid,
● glycolic acid,
● hydrabramine,
● lactic acid,
● L-arginine,
● L-lysine,
● naphtoic acids,
● *N*-methyl-glucamine,
● piperazine
● succinic acid,
● tartaric acid,
● tromethamine, or
● vanillic acid;
more preferably is selected from
● 1-(2-hydroxyethyl)-pyrrolidine, or
● 2-(Diethylamino)-ethanol,
● 4-(2-hydroxyethyl)-morpholine,
● benethamine,
● benzathine,
● benzoic acid,
● benzylamine,
● betaine (trimethylglycine),
● choline,
● citric acid,
● D-alanine,
● deanol,
● diethylamine,
● gentisic acid,
● glycolic acid,
● hydrabramine,
● lactic acid,
● L-arginine,
● L-lysine,
● naphtoic acids,
● *N*-methyl-glucamine,
● succinic acid,
● tartaric acid,
● tromethamine, or
● vanillic acid;
most preferably is selected from
● 1-(2-hydroxyethyl)-pyrrolidine, or
● 2-(diethylamino)-ethanol,
● 4-(2-hydroxyethyl)-morpholine,
● benethamine,
● benzathine,
● benzoic acid,
● benzylamine,
● citric acid,
● deanol,
● diethylamine,
● gentisic acid,
● glycolic acid,
● hydrabramine,
● lactic acid,
● naphtoic acids,
● N-methyl-glucamine,
● succinic acid,
● tartaric acid,
● tromethamine, or
● vanillic acid;
● or - optionally - D-alanine.

In an alternative preferred embodiment of the joined mixture according to the invention the organic molecule is selected from
● benethamine,
● benzathine,
● benzylamine,
● N-methyl-glucamine,
● 4-(2-hydroxyethyl)-morpholine
● 1-(2-hydroxyethyl)-pyrrolidine,
● benzoic acid,
● citric acid,
● D-aminoacids, like D-alanine,
● gentisic acid,
● glycolic acid,
● lactic acid, preferably L-lactic acid,
● naphtoic acids, like 1-hydroxy-2-naphtoic acid,
● succinic acid,
● tartaric acid, preferably L-tartaric acid; or
● vanillic acid,

In an alternative preferred embodiment of the joined mixture according to the invention the organic molecule is selected from
● benethamine,
● benzathine,
● benzylamine,
● N-methyl-glucamine,
● 4-(2-hydroxyethyl)-morpholine, or
● 1-(2-hydroxyethyl)-pyrrolidine,
preferably selected from
● benethamine,
● benzathine, or
● benzylamine.

In an alternative preferred embodiment of the joined mixture according to the invention the organic molecule is selected from
● benzoic acid,
● citric acid,
● D-aminoacids, like D-alanine,
● gentisic acid,
● glycolic acid,
● lactic acid, preferably L-lactic acid,
● naphtoic acids, like 1-hydroxy-2-naphtoic acid,
● succinic acid,
● tartaric acid, preferably L-tartaric acid; or
● vanillic acid,
preferably is gentisic acid.

In an alternative preferred embodiment of the joined mixture according to the invention the joined mixture is a hydrate or solvate.

In an alternative preferred embodiment of the joined mixture according to the invention the joint mixture is present in crystalline form. In a preferred embodiment this joint mixture is a polymorph of this crystalline form.

In an alternative preferred embodiment of the joined mixture according to the invention the joint mixture is in amorphous form.

A further aspect of the invention refers to a pharmaceutical formulation comprising at least one joint mixture according to the invention and optionally at least one physiologically acceptable excipient.

A further aspect of the invention refers to a joint mixture according to the invention for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy. An alternative aspect of the invention refers to the use of a joint mixture according to the invention in the production of a medicament or pharmaceutical formulation for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or for use in the regulation of HDAC6 activity, the modulation of stem cells, the prevention of grey hair, the promotion of neuronal growth, the prevention of bone and joint diseases, or as adjuvant in cancer therapy.

For the medicament or pharmaceutical formulation according to the present invention or for a medicament or pharmaceutical formulation used in the use of the joint mixture according to the invention the medicament or pharmaceutical formulation can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical formulation of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intramuscularly, intraperitoneally, or intravenously.

Medicaments or pharmaceutical formulation according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical formulation according to the present invention may also comprise an enteric coating, so that their dissolution is dependent on pH-value. Due to said coating the medicament may pass the stomach undissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more of the salts or their crystalline form as defined herein and 40-99 % by weight of one or more auxiliary substances (additives/excipients).

The joint mixture of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 10 to 2000 milligrams of active substance (joined mixture according to the invention) to be administered during one or several intakes per day.

In a very preferred embodiment the medicament or pharmaceutical formulation of the invention is an oral pharmaceutical composition. Very suitable oral pharmaceutical compositions comprising 4-phenyl-butyric acid or a physiologically acceptable salt thereof are disclosed in EP1427396 (WO2003/22253), the content of which forming herewith part of the description/disclosure of the current invention. Accordingly, it is very preferred if such an oral pharmaceutical composition comprising a joint mixture according to the invention is formulated in an analoguous way to the formulation presented in EP1427396 (W02003/22253). It is thus further very preferred if an oral medicament according to the invention especially a pharmaceutical composition comprises 100 to 2000 mg of the joined mixture according to the invention, preferably 200 to 1000 mg, most preferably approximately 500 mg or 250 mg of the joined mixture according to the invention.

In another alternative embodiment the joint mixture is formulated in a medicament or pharmaceutical formulation in form of a tablet wherein each gram of granulate contains 100 to 2000 mg of the joined mixture according to the invention, preferably 500 to 1000 mg of the joined mixture according to the invention. Preferably the granulate does further comprise microcrystalline cellulose, magnesium stearate and/or colloidal anhydrous silica.

In another alternative embodiment the joint mixture is formulated in a medicament or pharmaceutical formulation in form of a granulate containing 100 to 2000 mg of the joined mixture according to the invention, preferably 500 to 1000 mg of the joined mixture according to the invention. Preferably the granulate does further comprise calcium stearate and/or colloidal anhydrous silica.

Another aspect of the invention refers to a process for the production of a joint mixture according to the invention being either a salt or a co-crystal in which 4-phenyl-butyric acid with the following steps:
a) 4-phenyl-butyric acid is dissolved in organic solvent 1 to form solution 1;
b) the organic molecule is dissolved in organic solvent 2 to form solution 2;
c) solution 1 and solution 2 are mixed;
d) in a first final step the organic solvents are removed from the mixture from step c) and a residue/joined mixture obtained.

In an embodiment of the process according to the invention the following steps are added after step d):
e) the residue from step d) is dissolved in organic solvent 3;
f) the resulting mixture is stirred while the temperature is raised above room temperature followed by lowering of the temperature;
g) in a last final step the organic solvent is removed from the mixture from step f) and the joined mixture obtained,

In a preferred embodiment of the process according to the invention:
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step f) is raised to 25 to 35°C, preferably to approximately 30°C; and/or
● where applicable - the temperature in step f) is lowered to below 25°C, preferably to approximately 20°C; and/or
● in the first and/or the last final step the organic solvent is removed under gas flow.

"THF" is Tetrahydrofuran; "EtOH" is ethanol; "MeCN" is cyanomethan/acetonitril; "TBME" is tert.-butyl-methyl ether; and "EtOAc" is ethyl acetate.

In a very preferred embodiment and aspect of the invention, the joint mixture according to the invention is a salt of 4-phenyl-butyric acid with a salt-forming organic molecule. In one embodiment the salt of 4-phenyl-butyric acid with the salt-forming organic molecule is in amorphous or crystalline form, preferably is in crystalline form.

"Salt-forming organic molecule" as used herein is defined as any organic molecule, preferably an amine, amino-acid or heterocycle, preferably an amine, which is able to become ionized and with which phenyl butyric acid or its derivative is able to form a salt.

In an embodiment of the joined mixture according to the invention being a salt is an organic salt according to general formula I 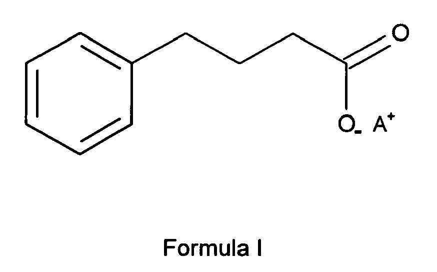 , wherein
A⁺ is selected from positively charged ions of a salt-forming organic molecule.

In a further embodiment this joint mixture according to the invention being a salt is a hydrate or solvate of a salt of 4-phenyl-butyric acid with a salt-forming organic molecule.

"Hydrate" is defined as a substance/joint mixture according to the invention containing water. Thus, it may be a crystalline salt with one or more water molecules in the crystalline structure or also amorphous containing water molecules.

"Solvate" is defined as a substance/joint mixture according to the invention containing a further solvent. One most prominent example of the solvents is an alcohol like ethanol or methanol thus leading to methanolates or ethanolates. Thus, it may be a crystalline salt with one or more alcohol molecules in the crystalline structure or also an amorphous form containing alcohol molecules.

In another embodiment the joint mixture according to the invention being a salt is a polymorph of a salt of 4-phenyl-butyric acid with a salt-forming organic molecule or of its hydrate or solvate.

In a very preferred embodiment and aspect of the invention in this salt of 4-phenyl-butyric acid with a salt-forming organic molecule according to the invention, the salt-forming organic molecule is selected from
● benethamine,
● benzathine,
● benzylamine,
● N-methyl-glucamine,
● 4-(2-hydroxyethyl)-morpholine, or
● 1-(2-hydroxyethyl)-pyrrolidine.

In a very preferred embodiment and aspect of the invention in this salt of 4-phenyl-butyric acid with a salt-forming organic molecule according to the invention, the salt-forming organic molecule is a molecule according to general formula II 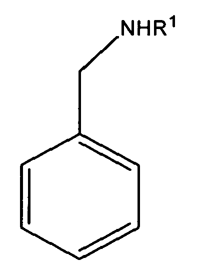 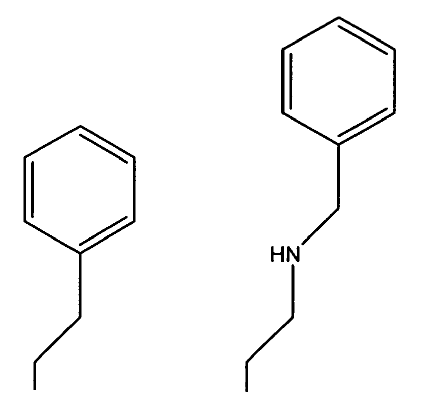 wherein R¹ is selected from H, 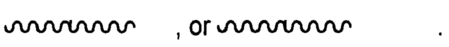 In one embodiment the salt of 4-phenyl-butyric acid with the salt-forming organic molecule according to general formula II is in amorphous or crystalline form, preferably is in crystalline form.

In a very preferred embodiment and aspect of the invention, in this salt of 4-phenyl-butyric acid with a salt-forming organic molecule according to the invention, the salt-forming organic molecule according to general formula II is benzylamine. In another embodiment the salt of 4-phenyl-butyric acid with benzylamine is in amorphous or crystalline form. In another preferred embodiment the salt of 4-phenyl-butyric acid with benzylamine is containing at least one water molecule thus being a "hydrate" (see above). In another preferred embodiment the salt of 4-phenyl-butyric acid with benzylamine is containing a solvent molecule thus being a "solvate" (see above). In another preferred embodiment the salt of 4-phenyl-butyric acid with benzylamine is existing in form of a polymorph of any of the salts, solvates, or hydrates.

"Benzylamine" (C₆H₅CH₂NH₂) is a colourless liquid with a melting point of -30°C, a mass weight of 107.15 g/mol of and has the general formula:

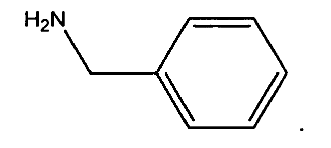

In a preferred embodiment the salt or crystalline form of the salt of 4-phenyl-butyric acid with benzylamine shows a FT-Raman pattern with peaks at 1031, 1002, 621, 487, 392, 219, and 196 [cm⁻¹].

In a preferred embodiment the salt or crystalline form of the salt of 4-phenyl-butyric acid with benzylamine shows a FT-Raman pattern with peaks at 1606, 1206, 1031, 1002, 941, 811, 797, 753, 746, 621, 487, 392, 270, 219, and 196 [cm⁻¹].

In a very preferred embodiment the salt or crystalline form of the salt of 4-phenyl-butyric acid with benzylamine shows a FT-Raman pattern with peaks at 1606, 1585, 1437, 1406, 1300, 1221, 1206, 1185, 1159, 1031, 1002, 941, 904, 811, 797, 753, 746, 667, 621, 571, 487, 392, 270, 219, and 196 [cm⁻¹].

Another aspect of the invention refers to a process for the production of a salt of 4-phenyl-butyric acid with benzylamine with the following steps:
a) 4-phenyl-butyric acid is dissolved in organic solvent 1 to form solution 1;
b) benzylamine is dissolved in organic solvent 2 to form solution 2;
c) solution 1 and solution 2 are mixed;
d) in a first final step the organic solvents are removed from the mixture from step c) and a residue/salt obtained.

Optionally, the following steps are added after step d):
e) the residue from step d) is dissolved in organic solvent 3;
f) the resulting mixture is stirred while the temperature is raised above room temperature followed by lowering of the temperature;
g) in a last final step the organic solvent is removed from the mixture from step f) and the salt is obtained.

Preferably, in that process:
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step f) is raised to 25 to 35°C, preferably to approximately 30°C; and/or
● where applicable - the temperature in step f) is lowered to below 25°C, preferably to approximately 20°C; and/or
● in the first and/or the last final step the organic solvent is removed under gas flow.

In a very preferred embodiment and aspect of the invention, in this salt of 4-phenyl-butyric acid with a salt-forming organic molecule according to the invention, the salt-forming organic molecule according to general formula II is benethamine. Preferably, in this salt of 4-phenyl-butyric acid with benethamine the molecular ratio between 4-phenyl-butyric acid and benethamine is between 4:1 and 1:4, more preferably is between 2:1 and 1:2. Most preferred is a salt of 4-phenyl-butyric acid with benethamine according to the invention, wherein the molecular ratio between 4-phenyl-butyric acid and benethamine is 1:1. In another embodiment the salt of 4-phenyl-butyric acid with benethamine is in amorphous or crystalline form, preferably is in crystalline form. In another preferred embodiment the salt of 4-phenyl-butyric acid with benethamine is containing at least one water molecule thus being a "hydrate" (see above). In another preferred embodiment the salt of 4-phenyl-butyric acid with benethamine is containing a solvent molecule thus being a "solvate" (see above). In another preferred embodiment the salt of 4-phenyl-butyric acid with benethamine is existing in form of a polymorph of any of the salts, solvates, or hydrates.

"Benethamine" (Benzyl-phenethyl-amine) is an organic molecule with the following structure:

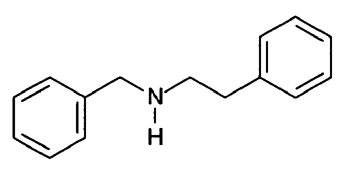

A preferred embodiment of the salt or crystalline form of the salt of 4-phenyl-butyric acid with benethamine according to the invention, with a molecular ratio between 4-phenyl-butyric acid and benethamine being 1:1 shows an X-Ray powder diffraction pattern with peaks [2θ] at 5.6, 16.9, 18.4, and 22.3 [°] with the 2θ values being obtained using copper radiation (Cu_{Kai} 1.54060A); or preferably at 5.58, 16.85, 18.39, and 22.25 [°]; or more preferably at 5.583, 16.854, 18.389, and 22.252 [°] with the 20 values being obtained using copper radiation (C_{UKa1} 1.54060A). All the 26 values are obtained using copper radiation (CU_{K}.₁ 1.54060A).

Another preferred embodiment of the salt or crystalline form of the salt of 4-phenyl-butyric acid with benethamine according to the invention, with a molecular ratio between 4-phenyl-butyric acid and benethamine being 1:1 shows an X-Ray powder diffraction pattern with peaks [2θ] at 5.6, 13.4, 14.6, 15.7, 15.8, 16.9, 18.4, 22.3, 22.4; 22.6, 23.7, 26.5, and 37.9 [°]; or preferably at 5.58, 13.39, 14.64, 15.68, 15.84, 16.85, 18.39, 22.25, 22.43, 22.57, 23.66, 26.48, and 37.85 [°]; or more preferably at 5.583, 13.385, 14.641, 15.684, 15.844, 16.854, 18.389, 22.252, 22.428, 22.567, 23.656, 26.484, and 37.850 [°]. All the 20 values are obtained using copper radiation (Cu_{Ka1} 1.54060A).

A very preferred embodiment of the salt or crystalline form of the salt of 4-phenyl-butyric acid with benethamine according to the invention, with a molecular ratio between 4-phenyl-butyric acid and benethamine being 1:1 shows an X-Ray powder diffraction pattern with peaks [20] at 5.6, 11.1, 13.4, 14.6, 15.7, 15.8, 16.9, 17.3, 18.0, 18.4, 19.0, 20.3, 21.3, 22.0, 22.3, 22.4, 22.6, 22.8, 22.9, 23.7, 24.1, 24.6, 24.8, 25.6, 26.5, 27.2, 27.9, 29.3, 30.1, 31.8, 32.0, 33.6, 35.0, and 37.9 [°];or preferably at 5.58, 11.15, 13.39, 14.64, 15.68, 15.84, 16.85, 17.27, 17.95, 18.39, 19.03, 20.34, 21.32, 22.05, 22.25, 22.43, 22.57, 22.78, 22.94, 23.66, 24.07, 24.65, 24.83, 25.61, 26.48, 27.15, 27.90, 29.28, 30.11, 31.75, 32.04, 33.62, 34.97, and 37.85 [°]; or more preferably at 5.583, 11.145, 13.385, 14.641, 15.684, 15.844, 16.854, 17.269, 17.954, 18.389, 19.030, 20.340, 21.316, 22.049, 22.252, 22.428, 22.567, 22.784, 22.943, 23.656, 24.070, 24.648, 24.826, 25.606, 26.484, 27.150, 27.896, 29.283, 30.105, 31.751, 32.038, 33.619, 34.967, and 37.850 [°]. All the 20 values are obtained using copper radiation (CU_{Ka1} 1.54060A).

A preferred embodiment of the salt or crystalline form of the salt of 4-phenyl-butyric acid with benethamine according to the invention, with a molecular ratio between 4-phenyl-butyric acid and benethamine being 1:1 shows an X-Ray powder diffraction pattern with peaks [d values] at 15.82, 5.26, 4.82, and 3.99 [A], or preferably at 15.817, 5.256, 4.821, and 3.992 [A] or more preferably at 15.81738, 5.25620, 4.82092, and 3.99190 [A].

Another preferred embodiment of the salt or crystalline form of the salt of 4-phenyl-butyric acid with benethamine according to the invention, with a molecular ratio between 4-phenyl-butyric acid and benethamine being 1:1 shows an X-Ray powder diffraction pattern with peaks [d values] at 15.82, 6.61, 6.05, 5.65, 5.59, 5.26, 4.82, 3.99, 3.96, 3.94, 3.76, 3.36, and 2.38 [A]; or preferably at 15.817, 6.610, 6.046, 5.646, 5.589, 5.256, 4.821, 3.992, 3.961, 3.937, 3.758, 3.363, and 2.375 [A]; or more preferably at 15.81738, 6.60995, 6.04550, 5.64578, 5.58891, 5.25620, 4.82092, 3.99190, 3.96095, 3.93692, 3.75796, 3.36279, and 2.37505 [A].

A very preferred embodiment of the salt or crystalline form of the salt of 4-phenyl-butyric acid with benethamine according to the invention, with a molecular ratio between 4-phenyl-butyric acid and benethamine being 1:1 shows an X-Ray powder diffraction pattern with peaks [d values] at 15.82, 7.93, 6.61, 6.05, 5.65, 5.59, 5.26, 5.13, 4.94, 4.82, 4.66, 4.36, 4.16, 4.03, 3.99, 3.96, 3.94, 3.90, 3.87, 3.76, 3.69, 3.61, 3.58, 3.48, 3.36, 3.28, 3.20, 3.05, 2.97, 2.82, 2.79, 2.66, 2.56, and 2.38 [A]; or preferably at 15.817, 7.933, 6.610, 6.046, 5.646, 5.589, 5.256, 5.131, 4.937, 4.821, 4.660, 4.362, 4.165, 4.028, 3.992, 3.961, 3.937, 3.900, 3.873, 3.758, 3.694, 3.609, 3.583, 3.476, 3.363, 3.282, 3.196, 3.047, 2.966, 2.816, 2.791, 2.663, 2.564, and 2.375 [A]; or more preferably at 15.81738, 7.93278, 6.60995, 6.04550, 5.64578, 5.58891, 5.25620, 5.13099, 4.93655, 4.82092, 4.65987, 4.36249, 4.16493, 4.02824, 3.99190, 3.96095, 3.93692, 3.89979, 3.87318, 3.75796, 3.69432, 3.60897, 3.58349, 3.47608, 3.36279, 3.28185, 3.19569, 3.04745, 2.96604, 2.81599, 2.79142, 2.66363, 2.56397, and 2.37505 [A].

A preferred embodiment of the salt or crystalline form of the salt of 4-phenyl-butyric acid with benethamine according to the invention, with a molecular ratio between 4-phenyl-butyric acid and benethamine being 1:1 shows a FT-Raman pattern with peaks at 3057, 3044, 1605, 1031, and 1003 [cm⁻¹]; or preferably at 3057, 3044, 2931, 2899, 2868, 1605, 1587, 1031, 1003, and 219 [cm⁻¹]; or more preferably at 3169, 3057, 3044, 3006, 2982, 2966, 2931, 2899, 2868, 1605, 1587, 1476, 1451, 1435, 1399, 1356, 1299, 1267, 1208, 1190, 1180, 1165, 1063, 1031, 1003, 944, 824, 754, 621, 581, 515, 408, 341, and 219 [cm-¹].

In a preferred embodiment of the salt or crystalline form of the salt of 4-phenyl-butyric acid with benethamine according to the invention, with a molecular ratio between 4-phenyl-butyric acid and benethamine being 1:1 the endothermic peak corresponding to the melting point has an onset at 80°C.

Another aspect of the invention refers to a process for the production of a salt of 4-phenyl-butyric acid with benethamine with the following steps:
a) 4-phenyl-butyric acid is dissolved in organic solvent 1 to form solution 1;
b) benethamine is dissolved in organic solvent 2 to form solution 2;
c) solution 1 and solution 2 are mixed;
d) in a first final step the organic solvents are removed from the mixture from step c) and a residue/salt obtained.

Optionally, the following steps are added after step d):
e) the residue from step d) is dissolved in organic solvent 3;
f) the resulting mixture is stirred while the temperature is raised above room temperature followed by lowering of the temperature;
g) in a last final step the organic solvent is removed from the mixture from step f) and the salt is obtained.

Preferably, in that process:
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step f) is raised to 25 to 35°C, preferably to approximately 30°C; and/or
● where applicable - the temperature in step f) is lowered to below 25°C, preferably to approximately 20°C; and/or
● in the first and/or the last final step the organic solvent is removed under gas flow.

Most preferably, in that process the organic solvents 1 and 2 are the same and are MeCN.

In a very preferred embodiment and aspect of the invention, in this salt of 4-phenyl-butyric acid with a salt-forming organic molecule according to the invention, the salt-forming organic molecule according to general formula II is benzathine. Preferably, in this salt of 4-phenyl-butyric acid with benzathine the molecular ratio between 4-phenyl-butyric acid and benzathine is between 4:1 and 1:4, more preferably is between 3:1 and 1:1. Most preferred is a salt of 4-phenyl-butyric acid with benzathine according to the invention, wherein the molecular ratio between 4-phenyl-butyric acid and benzathine is 2:1. In another preferred embodiment the salt of 4-phenyl-butyric acid with benzathine is hydrated, is a hydrate, e.g. mono-hydrate, di-hydrate etc. In another embodiment the salt of 4-phenyl-butyric acid with benzathine is in amorphous or crystalline form, preferably is in crystalline form. In another preferred embodiment the salt of 4-phenyl-butyric acid with benzathine is containing a solvent molecule thus being a "solvate" (see above). In another preferred embodiment the salt of 4-phenyl-butyric acid with benzathine is containing a solvent molecule thus being a "solvate" (see above) or is hydrated, is a hydrate, e.g. mono-hydrate, di-hydrate etc., preferably is a hydrate. In another preferred embodiment the salt of 4-phenyl-butyric acid with benzathine is existing in form of a polymorph of any of the salts, solvates, or hydrates.

Benzathine (N,N'-Dibenzyl-ethane-1,2-diamine) is an organic molecule with the following structure:

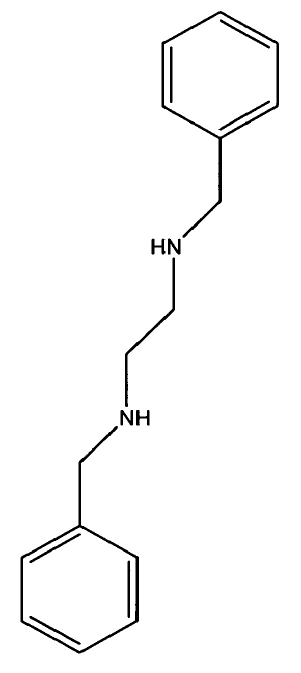

A preferred embodiment of the salt or crystalline form of the salt of 4-phenyl-butyric acid with benzathine according to the invention, with a molecular ratio between 4-phenyl-butyric acid and benzathine being 2:1 shows an X-Ray powder diffraction pattern with peaks [20] at 5.2 and 15.8 [°]; preferably at 5.24, and 15.84 [°]; more preferably at 5.241, and 15.837 [°]. All the 20 values are obtained using copper radiation (CuKai 1.54060A).

Another preferred embodiment of the salt or crystalline form of the salt of 4-phenyl-butyric acid with benzathine according to the invention, with a molecular ratio between 4-phenyl-butyric acid and benzathine being 2:1 shows an X-Ray powder diffraction pattern with peaks [2θ] at 5.2, 15.8, 19.0, 21.0, 21.2, and 22.5 [°]; preferably at 5.24, 15.84, 19.02, 21.04, 21.20, and 22.49 [°]; more preferably at 5.241, 15.837, 19.019, 21.042, 21.200, and 22.485, [°]. All the 20 values are obtained using copper radiation (Cu_{Ka1} 1.54060A).

A very preferred embodiment of the salt or crystalline form of the salt of 4-phenyl-butyric acid with benzathine according to the invention, with a molecular ratio between 4-phenyl-butyric acid and benzathine being 2:1 shows an X-Ray powder diffraction pattern with peaks [20] at 5.2, 10.5, 13.1, 14.9, 15.8, 16.9, 17.4, 18.2, 19.0, 19.7, 20.2, 21.0, 21.2, 22.5, 23.6, 24.1, 25.3, 26.1, 27.4, 28.2, 28.6, 31.2, 32.0, 32.3, 32.8, 33.6, 35.0, 36.4, 37.5, and 38.6 [°]; 5.24, 10.54, 13.12, 14.88, 15.84, 16.86, 17.40, 18.17, 19.02, 19.66, 20.18, 21.04, 21.20, 22.49, 23.60, 24.07, 25.28, 26.12, 27.35, 28.16, 28.62, 31.17, 32.01, 32.26, 32.80, 33.57, 34.96, 36.36, 37.52, and 38.62 [°]; 5.241, 10.541, 13.117, 14.878, 15.837, 16.857, 17.402, 18.171, 19.019, 19.657, 20.177, 21.042, 21.200, 22.485, 23.601, 24.069, 25.275, 26.121, 27.352, 28.160, 28.624, 31.174, 32.008, 32.263, 32.796, 33.565, 34.964, 36.362, 37.518, and 38.618 [°].All the 2θ values are obtained using copper radiation (CU_{Ka1} 1.54060A).

A preferred embodiment of the salt or crystalline form of the salt of 4-phenyl-butyric acid with benzathine according to the invention, with a molecular ratio between 4-phenyl-butyric acid and benzathine being 2:1 shows an X-Ray powder diffraction pattern with peaks [d-values] at 15.82, 5.26, 4.82, and 3.99 [A]; preferably at 15.817, 5.256, 4.821, and 3.992 [A]; more preferably at 15.81738, 5.25620, 4.82092, and 3.99190 [A].

A very preferred embodiment of the salt or crystalline form of the salt of 4-phenyl-butyric acid with benzathine according to the invention, with a molecular ratio between 4-phenyl-butyric acid and benzathine being 2:1 shows an X-Ray powder diffraction pattern with peaks [d-values] at 16.85, 5.59, 4.66, 4.22, 4.19, and 3.95 [A]; preferably at 16.849, 5.592, 4.663, 4.219, 4.188, and 3.951 [A]; more preferably at 16.8488, 5.5915, 4.6626, 4.2186, 4.1876, and 3.9511 [A].

A very preferred embodiment of the salt or crystalline form of the salt of 4-phenyl-butyric acid with benzathine according to the invention, with a molecular ratio between 4-phenyl-butyric acid and benzathine being 2:1 shows an X-Ray powder diffraction pattern with peaks [d-values] at 16.85, 8.39, 6.74, 5.95, 5.59, 5.26, 5.09, 4.88, 4.66, 4.51, 4.40, 4.22, 4.19, 3.95, 3.77, 3.69, 3.52, 3.41, 3.26, 3.17, 3.12, 2.87, 2.79, 2.77, 2.73, 2.67, 2.56, 2.47, 2.40, and 2.33 [A]; preferably at 16.849, 8.386, 6.744, 5.950, 5.592, 5.255, 5.092, 4.878, 4.663, 4.513, 4.397, 4.219, 4.188, 3.951, 3.767, 3.695, 3.521, 3.409, 3.258, 3.166, 3.116, 2.867, 2.794, 2.773, 2.729, 2.668, 2.564, 2.469, 2.395, and 2.330 [A]; more preferably at 16.8488, 8.3855, 6.7442, 5.9498, 5.5915, 5.2553, 5.0920, 4.8782, 4.6626, 4.5127, 4.3974, 4.2186, 4.1876, 3.9511, 3.7667, 3.6945, 3.5208, 3.4087, 3.2581, 3.1664, 3.1161, 2.8668, 2.7940, 2.7725, 2.7286, 2.6678, 2.5642, 2.4687, 2.3953, and 2.3296 [A].

A preferred embodiment of the salt or crystalline form of the salt of 4-phenyl-butyric acid with benzathine according to the invention, with a molecular ratio between 4-phenyl-butyric acid and benzathine being 2:1 shows a FT-Raman pattern with peaks at 3063, 3054, 1003, and 193 [cm-¹], or preferably at 3063, 3054, 2960, 2915, 2856, 1605, 1205, 1043, 1036, 1003, and 193 [cm⁻¹]; or more preferably at 3063, 3054, 3036, 3001, 2960, 2915, 2890, 2856, 2691, 1605, 1585, 1471, 1454, 1438, 1407, 1378, 1314, 1289, 1232, 1205, 1178, 1165, 1155, 1081, 1064, 1043, 1036, 1003, 954, 934, 908, 817, 787, 741, 670, 622, 574, 499, 481, 456, 378, 338, 265, and 193 [cm⁻¹].

In a preferred embodiment of the salt or crystalline form of the salt of 4-phenyl-butyric acid with benzathine according to the invention, with a molecular ratio between 4-phenyl-butyric acid and benzathine being 2:1 the endothermic peak corresponding to the melting point has an onset at 74°C.

Another aspect of the invention refers to a process for the production of a salt of 4-phenyl-butyric acid with benzathine with the following steps:
a) 4-phenyl-butyric acid is dissolved in organic solvent 1 to form solution 1;
b) benzathine is dissolved in organic solvent 2 to form solution 2;
c) solution 1 and solution 2 are mixed;
d) in a first final step the organic solvents are removed from the mixture from step c) and a residue/salt obtained.

Optionally, the following steps are added after step d):
e) the residue from step d) is dissolved in organic solvent 3;
f) the resulting mixture is stirred, while the temperature is raised above room temperature followed by lowering of the temperature;
g) in a last final step the organic solvent is removed from the mixture from step f) and the salt is obtained.

Preferably, in that process:
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step f) is raised to 25 to 35°C, preferably to approximately 30°C; and/or
● where applicable - the temperature in step f) is lowered to below 25°C, preferably to approximately 20°C; and/or
● in the first and/or the last final step the organic solvent is removed under gas flow.

Most preferably, in that process the organic solvents 1 and 2 are the same and are MeCN.

Another aspect of the invention refers to a pharmaceutical formulation comprising a salt or crystalline form thereof according to the invention being either generally a salt of 4-phenyl-butyric acid with a salt-forming organic molecule, e.g. according to formulas I or II, or a salt of 4-phenyl-butyric acid with a salt-forming organic molecule being benzylamine, or a salt of 4-phenyl-butyric acid with a salt-forming organic molecule being benethamine, or a salt of 4-phenyl-butyric acid with a salt-forming organic molecule being benzathine, respectively and optionally at least one physiologically acceptable excipient.

Salts or crystalline forms thereof according to the invention being either generally a salt of 4-phenyl-butyric acid with a salt-forming organic molecule, e.g. according to formulas I or II, , or a salt of 4-phenyl-butyric acid with a salt-forming organic molecule being benzylamine, or a salt of 4-phenyl-butyric acid with a salt-forming organic molecule being benethamine, or a salt of 4-phenyl-butyric acid with a salt-forming organic molecule being benzathine, respectively for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.

In a very preferred embodiment and aspect of the invention, the joint mixture according to the invention is a co-crystal of 4-phenyl-butyric acid (or derivative or salt thereof) with a co-crystal-forming organic molecule. In an embodiment the molecular ratio between 4-phenyl-butyric acid (or anion thereof) and co-crystal forming organic molecule is between 4:1 and 1:4. In another embodiment the co-crystal does contain at least one further molecule being selected from water (a hydrate), solvents like alcohols (methanol, ethanol) (a solvate or alcoholate) or a cation. In an embodiment, any of these co-crystals according to the invention is existing in form of a polymorph.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction, which is neither ionic nor covalent and includes for example: hydrogen bonds, van der Waals forces, and S-S-interactions. Solvates or salts of phenyl butyric acid and an organic compound that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In the scientific literature there is currently some discussion on the proper use of the word co-crystal (see for example Desiraju, CrystEngComm, 2003, 5(82), 466-467 and Dunitz, CrystEngComm, 2003, 5(91 ), 506). A recent article by Zaworotko (Zaworotko, Crystal Growth & Design, Vol. 7, No. 1, 2007, 4-9) gives a definition of co-crystal which is in line with the definition given above.

"Co-crystal forming organic molecule" as used herein is defined as any organic molecule, preferably an organic acid, preferably a carboxylic acid with which phenyl butyric acid or its salt is able to form co-crystals.

In a very preferred embodiment and aspect of the invention in this co-crystal of 4-phenyl-butyric acid with a co-crystal-forming organic molecule according to the invention, the co-crystal-forming-forming organic molecule is an organic acid, preferably an organic acid selected from
● benzoic acid,
● citric acid,
● gentisic acid,
● glycolic acid,
● lactic acid, preferably L-lactic acid,
● naphtoic acid, 1-hydroxy-2-,
● succinic acid,
● tartaric acid,
● vanillic acid, preferably L-tartaric acid, or
● a D-amino- acid, like D-alanine.

In a very preferred embodiment and aspect of the invention, in this co-crystal of 4-phenyl-butyric acid (or derivative or salt thereof) with a co-crystal-forming organic molecule according to the invention, the co-crystal-forming-forming organic molecule is an organic acid, preferably is gentisic acid. In an embodiment the molecular ratio between 4-phenyl-butyric acid (or anion thereof) and the gentisic acid is between 4:1 and 1:4. In another embodiment the co-crystal does contain at least one further molecule being selected from water (a hydrate), solvents like alcohols (methanol, ethanol) (a solvate or alcoholate) or a cation. In an embodiment, any of these co-crystals according to the invention is existing in form of a polymorph.

In a preferred embodiment the co-crystal of the of 4-phenyl-butyric acid with gentesic acid shows a FT-Raman pattern with peaks at 1326, 796, 754, 452, and 187 [cm⁻¹].

In another preferred embodiment the co-crystal of the of 4-phenyl-butyric acid with gentesic acid shows a FT-Raman pattern with peaks at 1326, 1003, 943, 796, 754, 560, 487, 452, 288, and 187 [cm⁻¹]_{.}

In a further preferred embodiment the co-crystal of the of 4-phenyl-butyric acid with gentesic acid shows a FT-Raman pattern with peaks at 1648, 1600, 1326, 1136, 1085, 1003, 943, 796, 754, 560, 487, 452, 382, 288, and 187 [cm⁻¹].

In a very preferred embodiment the co-crystal of the of 4-phenyl-butyric acid with gentesic acid shows a FT-Raman pattern with peaks at 1648, 1600, 1484, 1384, 1326, 1242, 1211, 1136, 1085, 1035, 1003, 943, 840, 796, 754, 703, 665, 622, 560, 487, 452, 382, 288, and 187 [cm⁻¹].

Another aspect of the invention refers to a pharmaceutical formulation comprising a co-crystal according to the invention being either generally a co-crystal of 4-phenyl-butyric acid (or derivative or salt thereof) with a co-crystal-forming organic molecule or a co-crystal of 4-phenyl-butyric acid (or derivative or salt thereof) with a co-crystal-forming organic molecule being gentisic acid, respectively and optionally at least one physiologically acceptable excipient.

Another aspect of the invention refers to a process for the production of a co-crystal according to the invention being a co-crystal of 4-phenyl-butyric acid (or derivative or salt thereof) with a co-crystal-forming organic molecule being gentisic acid with the following steps:
a) 4-phenyl-butyric acid is dissolved in organic solvent 1 to form solution 1;
b) the organic molecule is dissolved in organic solvent 2 to form solution 2;
c) solution 1 and solution 2 are mixed;
d) in a first final step the organic solvents are removed from the mixture from step c) and a residue/joined mixture obtained.

Optionally, the following steps are added after step d):
h) the residue from step d) is dissolved in organic solvent 3;
i) the resulting mixture is stirred while the temperature is raised above room temperature followed by lowering of the temperature;
j) in a last final step the organic solvent is removed from the mixture from step f) and the Co-crystal is obtained.

Preferably, in that process:
● the organic solvents 1 and 2 are the same organic solvent; and/or
● the organic solvents 1 and/or 2 is/are selected from EtOH, THF, or MeCN; and/or
● the organic solvent 3 is/are selected from toluene, TBME, heptane, or EtOAc; and/or
● where applicable - the temperature in step i) is raised to 25 to 35°C, preferably to approximately 30°C; and/or
● where applicable - the temperature in step i) is lowered to below 25°C, preferably to approximately 20°C; and/or
● in the first and/or the last final step the organic solvent is removed under gas flow.

Co-crystal according the invention being either generally a co-crystal of 4-phenyl-butyric acid (or derivative or salt thereof) with a co-crystal-forming organic molecule or a co-crystal of 4-phenyl-butyric acid (or derivative or salt thereof) with a co-crystal-forming organic molecule being gentisic acid, respectively for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.

The present invention is illustrated below with the help of the following figures and examples. These illustrations are given solely by way of example and do not limit the invention.

### FIGURES:

### Brief description of the figures:

**Figure 1****:** FTR-Raman spectrum showing the fitting spectra from two different phenylbutyrate-mono-benethamine-salts (molecular ratio phenylbutyrate : benethamine being 1 : 1) produced by different processes (first and second spectra from top) with the top being the production according to Example 1. They are different from either the spectrum of the free phenylbutyric acid (third from top) or of the benthamine salt-former (lowest), indicating a crystalline salt.
**Figure 2****:** Powder X-Ray diffraction pattern of phenylbutyrate-mono-benethamine-salt according to Example 1.
**Figure 3****:** ¹HNMR spectrum of the phenylbutyrate-mono-benethamine-salt according to Example 1.
**Figure 4****:** Optical microscopy pictures crystals of the phenylbutyrate-mono-benethamine-salt according to Example 1 (without crossed polarizers [left]; with crossed polarizers [right]).
**Figure 5****:** TG-FITR thermogram of the phenylbutyrate-mono-benethamine-salt according to Example 1.
**Figure 6****:** DSC thermogram of the phenylbutyrate-mono-benethamine-salt according to Example 1.
**Figure 7****:** DVS / hygroscopy measurement of the phenylbutyrate-mono-benethamine-salt according to Example 1.
**Figure 8****:** FTR-Raman spectrum showing the fitting spectra from two different phenylbutyrate-hemi-benzathine-salts (molecular ratio phenylbutyrate : benzathine being 2 : 1) produced by different processes (first and second spectra from top) with the top being the production according to Example 1. They are different from either the spectrum of the free phenylbutyric acid (third from top) or of the benzathine salt-former (lowest), indicating a crystalline salt.
**Figure 9****:** Powder X-Ray diffraction pattern of the phenylbutyrate-hemi-benzathine-salt according to Example 2.
**Figure 10****:** ¹HNMR spectrum of the phenylbutyrate-hemi-benzathine-salt according to Example 2.
**Figure 11**: Optical microscopy pictures crystals of the phenylbutyrate-hemi-benzathine-salt according to Example 2 (without crossed polarizers [left]; with crossed polarizers [right]).
**Figure 12****:** TG-FITR thermogram of the phenylbutyrate-hemi-benzathine-salt according to Example 2.
**Figure 13****:** DSC thermogram of the phenylbutyrate-hemi-benzathine-salt according to Example 2.
**Figure 14****:** DVS / hygroscopy measurement of the phenylbutyrate-hemi-benzathine-salt according to Example 2.
**Figure 15****:** FT-Raman spectrum of the residue (top) of the measurement of aqueous solubility of phenylbutyrate-hemi-benzathine-salt according to Example 2 compared to the spectra of the phenylbutyrate-hemi-benzathine-salt according to Example 2 (2^{nd} from top), the phenylbutyric acid (3^{rd} from top) and the salt-former benzathine (lowest).
**Figure 16****:** FT-Raman spectra of the phenylbutyrate-benzylamine-salt according to Example 3 (top) or the amorphous form of phenylbutyrate-benzylamine-salt according to Example 4 (second from top) compared to the spectra of phenylbutyric acid (3^{rd} from top) and benzylamine (lowest).
**Figure 17****:** FT-Raman spectra of the co-crystal of phenylbutyric acid and gentisic acid according to Example 5 (top) compared to the spectra of phenylbutyric acid (middle) and gentisic acid (lowest).

### EXAMPLES:

### Example 1: Production and characterization of 4-Phenvibutyrate-Mono-Benethamine salt

### Production:

1.0146 g of 4-phenylbutyrie acid were dissolved in 1.0 mL of MeCN. 1.28 mL of benethamine mixed with 1 mL of MeCN were slowly added. The clear, slightly yellow solution was stirred at room temperature overnight. A thick suspension was observed. 4.0 mL of MeCN were added to obtain a stirable suspension. This suspension was agitated and sonicated. The suspension was stirred at room temperature for 2 h. An aliquot was recovered by filter centrifugation (0.2-µm PTFE membrane). The solid was examined by FT-Raman spectroscopy and optical microscopy and the aliquot dried under vacuum for 5 min. The solid was examined by PXRD and ¹H-NMR. The remaining solid was recovered by vacuum filtration (pore size P4) and the solid dried at room temperature under vacuum over night. 1.7585 g of white solid were obtained. This corresponds to a 4-Phenylbutyrate-Mono-Benethamine salt in which the molecular ratio between 4-phenylbutyrate and benethamine is 1 : 1.

### Characterization:

The white solid was examined by
a) FT-Raman spectroscopy;
b) PXRD;
c) ¹H-NMR;
d) Elemental analysis
e) Optical microscopy;
f) TG-FTI R;
g) DSC; and
h) DVS; whereas finally
i) solubility was determined.

### a) FT-Raman Spectroscopy:

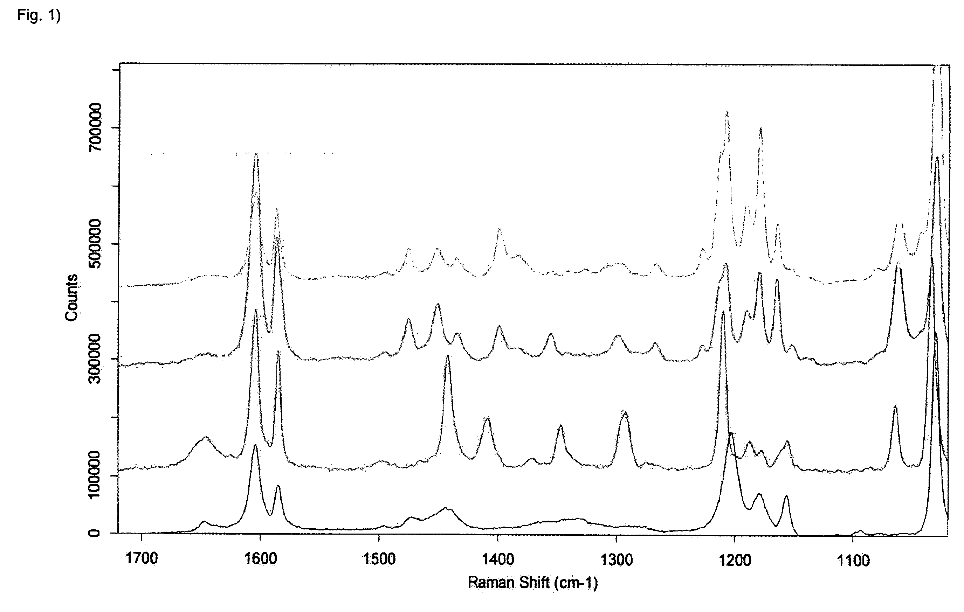

A Bruker RFS100 with OPUS 6.5 software was used having an Nd:YAG 1064-nm excitation and a Ge detector, 3500-100 cm⁻¹ range. The measurement conditions were 100-300 mW laser power, 64 - 128 scans, 2 cm⁻¹ resolution. The Raman spectrum (Fig. 1) shows spectra from two different phenylbutyrate-mono-benethamine-salts produced by different processes (first and second spectra from top; with the top being the product according to Example 1). Fig. 1) shows that the new salt from the two different experiments (first and second spectra from top) are different in spectrum from the spectrum of either the free phenylbutyric acid (third from top) or the benthamine salt-former (lowest), indicating a crystalline salt.

The raw data for the results show a FT-Raman pattern with peaks [cm⁻¹] at:

**Table 1 )**

| **peaks [cm**⁻¹**]** | **abs. int.** |
|---|---|
| 3169 | 0.02 |
| 3057 | 0.69 |
| 3044 | 0.31 |
| 3006 | 0.09 |
| 2982 | 0.12 |
| 2966 | 0.12 |
| 2931 | 0.16 |
| 2899 | 0.17 |
| 2868 | 0.15 |
| 1605 | 0.27 |
| 1587 | 0.17 |
| 1476 | 0.06 |
| 1451 | 0.08 |
| 1435 | 0.05 |
| 1399 | 0.05 |
| 1356 | 0.05 |
| 1299 | 0.04 |
| 1267 | 0.03 |
| 1208 | 0.14 |
| 1190 | 0.08 |
| 1180 | 0.12 |
| 1165 | 0.11 |
| 1063 | 0.14 |
| 1031 | 0.27 |
| 1003 | 0.97 |
| 944 | 0.03 |
| 824 | 0.14 |
| 754 | 0.03 |
| 621 | 0.12 |
| 581 | 0.03 |
| 515 | 0.03 |
| 408 | 0.02 |
| 341 | 0.03 |
| 219 | 0.22 |

### b) PXRD:

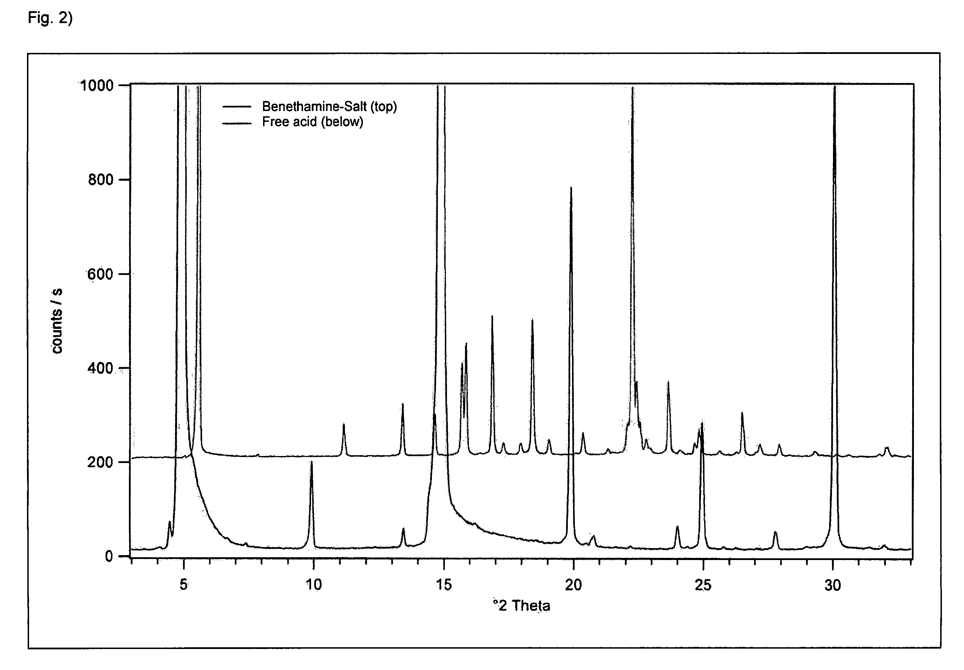

A Bruker D8 Advance with Cu-Ka radiation (CU_{Ka1} 1.54060A), and LynxEye detector was used. The measurement conditions were 40 kV/40 mA tube power; 0.02. 28 step size, 37 s step time, 2.550. 28 scanning range. The samples were measured under ambient air atmosphere without any special treatment other than the application of slight pressure to get a flat surface; silicon single crystal sample holder, 0.1-mm deep. The PXRD pattern (Fig. 2) of the new salt (top) differs from that of the free phenylbutyric acid (lower) and confirms the crystallinity of the new salt.

The raw data for the results show a PXRD pattern with peaks [20] [°] and d-Values [A] at:

**Table 2)**

| **Angle 2-** 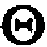 **°** | **d-Value A** | **Intensity %** |
|---|---|---|
| 5.583 | 15.81738 | 100.0 |
| 11.145 | 7.93278 | 4.5 |
| 13.385 | 6.60995 | 6.9 |
| 14.641 | 6.04550 | 6.2 |
| 15.684 | 5.64578 | 12.5 |
| 15.844 | 5.58891 | 14.6 |
| 16.854 | 5.25620 | 19.6 |
| 17.269 | 5.13099 | 2.3 |
| 17.954 | 4.93655 | 2.2 |
| 18.389 | 4.82092 | 17.8 |
| 19.030 | 4.65987 | 2.8 |
| 20.340 | 4.36249 | 3.7 |
| 21.316 | 4.16493 | 1.6 |
| 22.049 | 4.02824 | 4.8 |
| 22.252 | 3.99190 | 49.1 |
| 22.428 | 3.96095 | 10.2 |
| 22.567 | 3.93692 | 5.0 |
| 22.784 | 3.89979 | 2.8 |
| 22.943 | 3.87318 | 1.6 |
| 23.656 | 3.75796 | 10.7 |
| 24.070 | 3.69432 | 1.5 |
| 24.648 | 3.60897 | 2.5 |
| 24.826 | 3.58349 | 4.3 |
| 25.606 | 3.47608 | 1.4 |
| 26.484 | 3.36279 | 7.0 |
| 27.150 | 3.28185 | 2.3 |
| 27.896 | 3.19569 | 2.4 |
| 29.283 | 3.04745 | 1.4 |
| 30.105 | 2.96604 | 0.9 |
| 31.751 | 2.81599 | 1.0 |
| 32.038 | 2.79142 | 2.0 |
| 33.619 | 2.66363 | 1.0 |
| 34.967 | 2.56397 | 1.7 |
| 37.850 | 2.37505 | 6.4 |

### c)¹H-NMR:

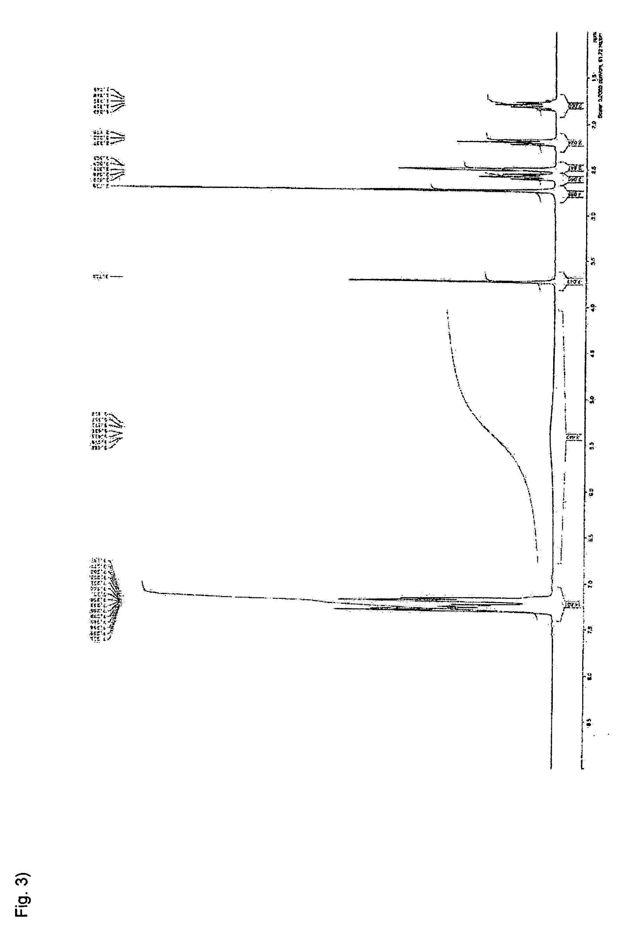

A Bruker DPX300 spectrometer with a proton frequency of 300.13 MHz, 30° excitation pulse, a recycle delay of 1 s at 300.13 MHz. 16 scans were accumulated and d₆-DMSO used as solvent. The ¹H-NMR spectrum (as well as the elemental analysis) agree with a 1:1 salt formation (Figure 3; as well as Table 3 below).

### d) Elemental Analysis:

A Leco CHN 800 was used for C, H, and N determination by combustion and a Leco RO-478 for determination of O by pyrolysis.

**Table 3): Elemental analysis of Example 1**

| | %C | %H | %N | %O | Σ |
|---|---|---|---|---|---|
| Example 1 (experimental determination) | 79.9 | 7.9 | 3.8 | 8.4 | 100.0 |
| C₁₀H₁₂O₂C₁₅H₁₇N (theoretical 1:1 salt) | 80.0 | 7.8 | 3.7 | 8.5 | 100.0 |
| Difference (experiment - theoretical) | -0.1 | 0.1 | 0.1 | - 0.1 | -- |

### e) Optical Microscopy:

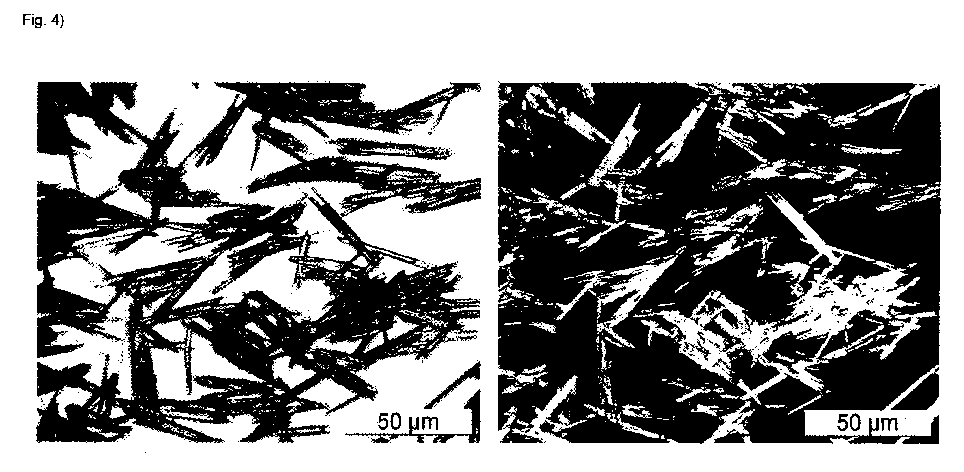

A Leitz Orthoplan 110680 microscope equipped with a Leica DFC280 camera and IM50 v.5 image-capturing software was used. Images were recorded with or without crossed polarizers and with 4x, 10x, or 25x magnification. Fig. 4) shows crystalline rods or thick needles (without crossed polarizers [left]; with crossed polarizers [right]).

### f) TG-FTIR:

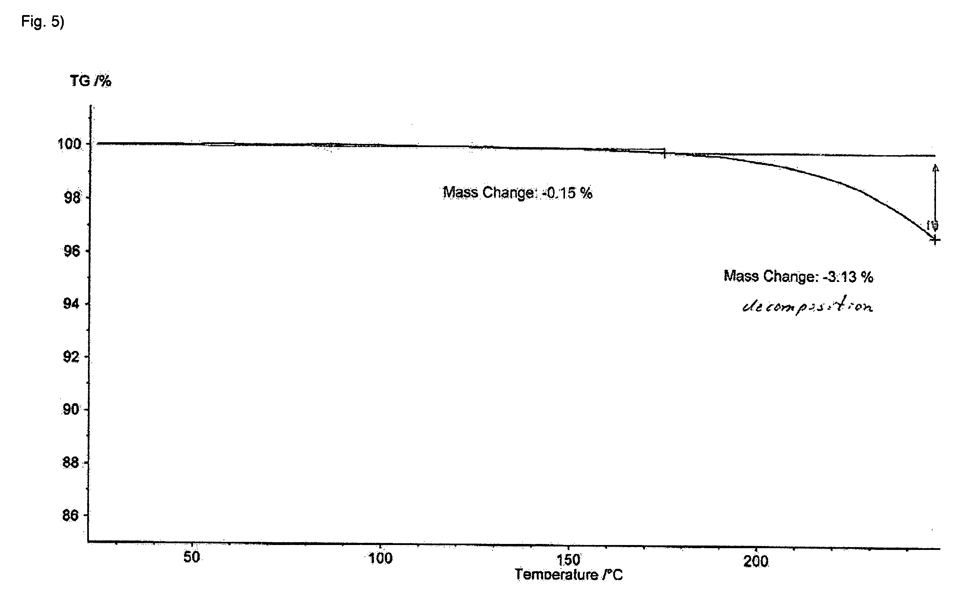

For the TG-FITR thermogram a Netzsch Thermo-Microbalance TG 209 with Bruker FT-IR Spectrometer Vector 22 and a Al crucible (with microhole) was used under N₂ atmosphere at 10 K/min heating rate in 25-250°C range. The TG-FTIR thermogram (Fig. 5) shows a mass loss of ∼0.15 wt.-% from 25-175°C which indicates that this salt is anhydrous. Further mass loss is observed at temperatures >175°C.

### g) DSC:

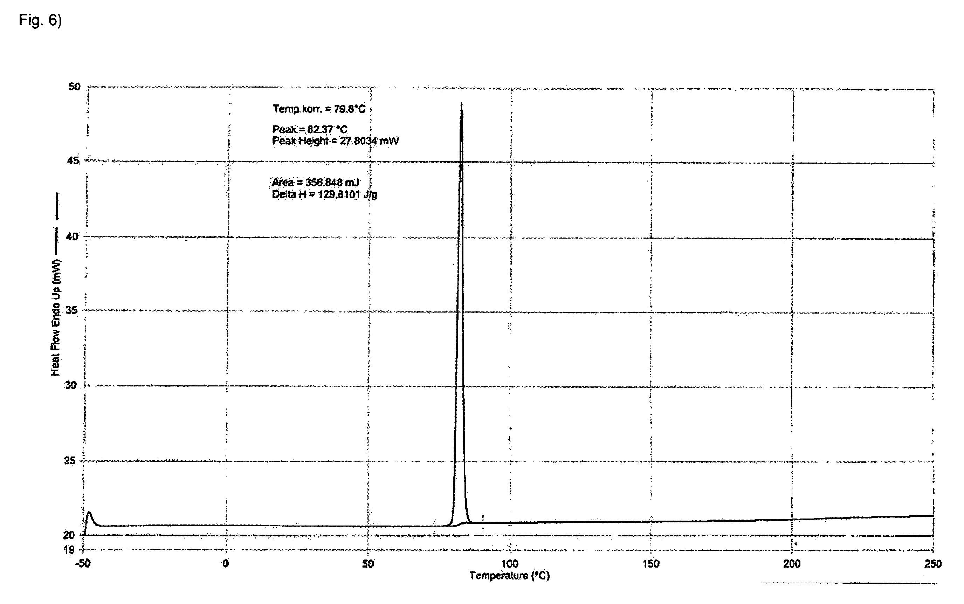

A Perkin Elmer DSC 7 with closed gold crucibles was used. The sample was filled and dried in an N₂ environment. The heating rate was 10 K/min between -50 to 250°C or 350°C range. The DSC thermogram (Figure 6) shows a melting endotherm at 80°C (ΔH = 129.8 J/g) and no further thermal event up to 250°C.

### h) DVS:

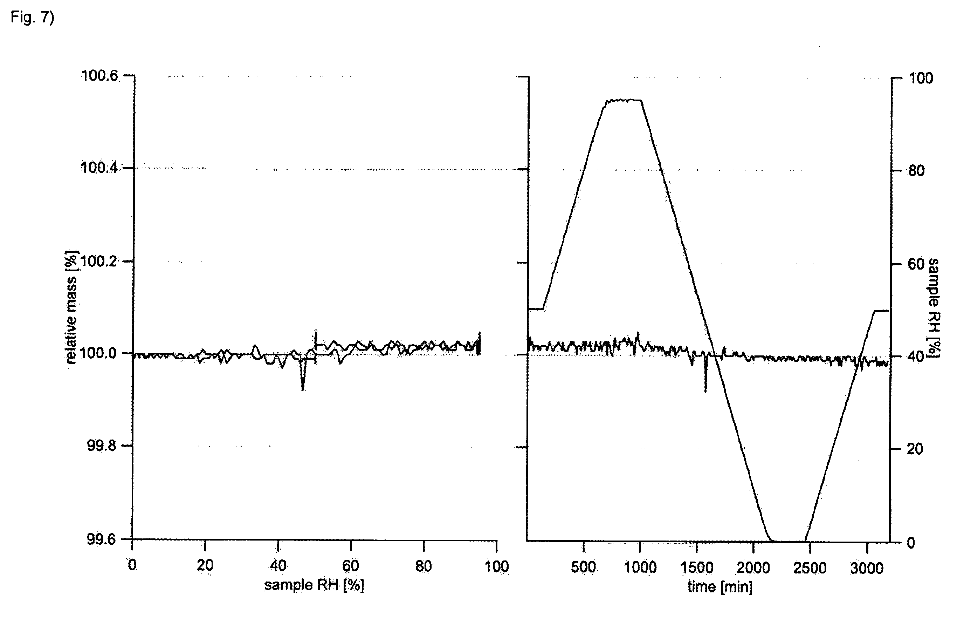

For the measurement of hygroscopy of the salt a Projekt Messtechnik Sorptions Prüfsystem SPS 11 - 100n was used. (Hygroscopy of the phenylbutyric acid for comparison was measured with the Surface Measurement Systems DVS-1 ). The salt sample was placed on an aluminium (SPS 11) holder (for phenylbutyric acid on a platinum holder (DVS-1)) on top of a microbalance and allowed to equilibrate for 2 h at 50% r.h. before starting the pre-defined humidity program:
(1)2hat50%r.h.
(2) 50 → 95% r.h. (5%/h); 5 h at 95% r.h.
(3) 95 → 0% r.h. (5%/h); 5 h at 0% r.h.
(4) 0 → 50% r.h. (5%/h); 2 h at 50% r.h.
The results are shown in Fig. 7). The salt was not hygroscopic. The FT-Raman spectrum of the sample after the DVS measurement shows no changes.

### i) Aqueous Solubility Determination:

For this measurement approximately 1.0 mL of doubly distilled water was added to 18 mg of the substance according Example 1 to be measured. The resulting suspension was equilibrated in a temperature-controlled *Eppendorf Thermomixer Comfort* shaker for 24 h at 25°C at a shaking rate of 600 rpm. The solid phase was recovered by filter centrifugation (0.10-µm PVDF membrane) and examined by FT-Raman spectroscopy. Concentrations in the filtrate (i.e., saturated solutions) were determined by HPLC (see below). The pH of the saturated solution was determined with a Metrohm 713 pH meter.
HPLC: *TSP* instrument with P4000 pump, AS3000 autosampler, SCM1000 degasser, UV3000 detector, and PC1000 Vers. 4.1 software.

**Table 4: HPLC method used for solubility determinations.**

| | | | |
|---|---|---|---|
| Column | Waters, XTerra MS C18, 100 mm x 4.6 mm, 5-µm particles (CC01) | | |
| Mobile phase A | H₂0/MeCN 95:5 +0.1% TFA | | |
| Mobile phase B | H₂0/MeCN 5:95 + 0.1 % TFA | | |
| Reference conc. | ca. 0.2 mg/mL | | |
| Retention time | 7.7-8.0 min | | |
| Isocratic | 0 min | 75% A | 25% B |
| | 15 min | 75% A | 25% B |
| Flow | 1.0mL/min | | |
| Injection volume | 10 µl | | |
| Wavelength | 207 nm | | |

An aqueous solubility (after 24 h of equilibration at 25°C) of 1.9 mg/mL was determined. The pH of the corresponding saturated solution was 7.2. The Raman spectrum of the solid residue was unchanged compared to the initial spectrum of the salt.

### Summary:

The 4-Phenylbutyrate-Mono-Benethamine salt having a molecular ratio of 1:1 is a crystalline anhydrous salt. It is clearly non-hygroscopic and is thus also advantageous in its own rights. In addition, the salt has with 80°C a significantly higher melting point than the phenylbutyric acid (51 °C). This salt also opens the path to develop an alternative to the known sodium phenylbutyrate avoiding sodium.

### Example 2: Production of 4-Phenylbutyrate-Hemi-Benzathine salt (1)

1.0053 g of 4-phenylbutyric acid were dissolved in 1.0 mL of MeCN. 1.4667 mL of benzathine mixed with 0.5 mL of MeCN are slowly added. The mixture is stirred at r.t. overnight and a clear solution is achieved. No precipitation is observed. The mixture is sonicated for 30 s and precipitation was observed. 8.0 mL of MeCN were added to obtain a stirable suspension which was agitated and sonicated. The suspension was stirred over night. An aliquot was recovered by filter centrifugation (0.2-µm PTFE membrane). The solid was examined by FT-Raman spectroscopy and optical microscopy. The aliquot was dried under vacuum for 5 min and the solid examined by PXRD and ¹H-NMR. The remaining solid was recovered by vacuum filtration (pore size P4) and the solid dried at room temperature under vacuum for 5 h. 0.8685 g of white solid were obtained. This corresponds to a 4-Phenylbutyrate-Mono-Benzathine salt in which the molecular ratio between 4-phenylbutyrate and benethamine is 2 : 1.

### Characterization:

The white solid was examined by
a) FT-Raman spectroscopy;
b) PXRD;
c) ¹H-NMR;
d) Elemental analysis
e) Optical microscopy;
f) TG-FTIR;
g) DSC; and
h) DVS; whereas finally
i) solubility was determined.

### a) FT-Raman Spectroscopy:

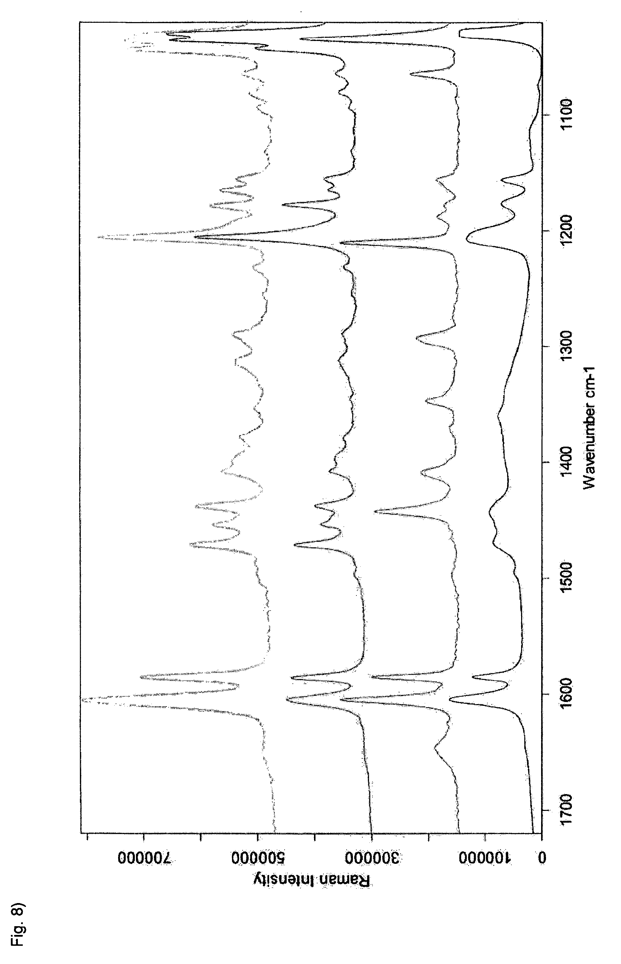

A Bruker RFS100 with OPUS 6.5 software was used having an Nd:YAG 1064-nm excitation and a Ge detector, 3500-100 cm⁻¹ range. The measurement conditions were 100-300 mW laser power, 64-128 scans, 2 cm⁻¹ resolution. The Raman spectrum (Fig. 8) shows spectra from two different phenylbutyrate-hemi-benzathine-salts produced by different processes (first and second spectra from top; with the top being the product according to Example 2). Fig. 8) shows that the new salt from the two different experiments (first and second spectra from top) are different in spectrum from the spectrum of either the free phenylbutyric acid (third from top) and the benzathine salt-former (lowest), indicating a crystalline salt..

The raw data for the results show a FT-Raman pattern with peaks [cm⁻¹] at:

**Table 5)**

| **peaks [cm**⁻¹**]** | **abs. int.** |
|---|---|
| 3063 | 0.48 |
| 3054 | 0.40 |
| 3036 | 0.14 |
| 3001 | 0.07 |
| 2960 | 0.17 |
| 2915 | 0.18 |
| 2890 | 0.14 |
| 2856 | 0.15 |
| 2691 | 0.02 |
| 1605 | 0.19 |
| 1585 | 0.14 |
| 1471 | 0.09 |
| 1454 | 0.07 |
| 1438 | 0.08 |
| 1407 | 0.06 |
| 1378 | 0.04 |
| 1314 | 0.04 |
| 1289 | 0.05 |
| 1232 | 0.02 |
| 1205 | 0.18 |
| 1178 | 0.07 |
| 1165 | 0.06 |
| 1155 | 0.04 |
| 1081 | 0.03 |
| 1064 | 0.04 |
| 1043 | 0.15 |
| 1036 | 0.15 |
| 1003 | 0.53 |
| 954 | 0.04 |
| 934 | 0.04 |
| 908 | 0.02 |
| 817 | 0.05 |
| 787 | 0.11 |
| 741 | 0.04 |
| 670 | 0.02 |
| 622 | 0.13 |
| 574 | 0.02 |
| 499 | 0.02 |
| 481 | 0.04 |
| 456 | 0.02 |
| 378 | 0.02 |
| 338 | 0.03 |
| 265 | 0.04 |
| 193 | 0.24 |

### b) PXRD:

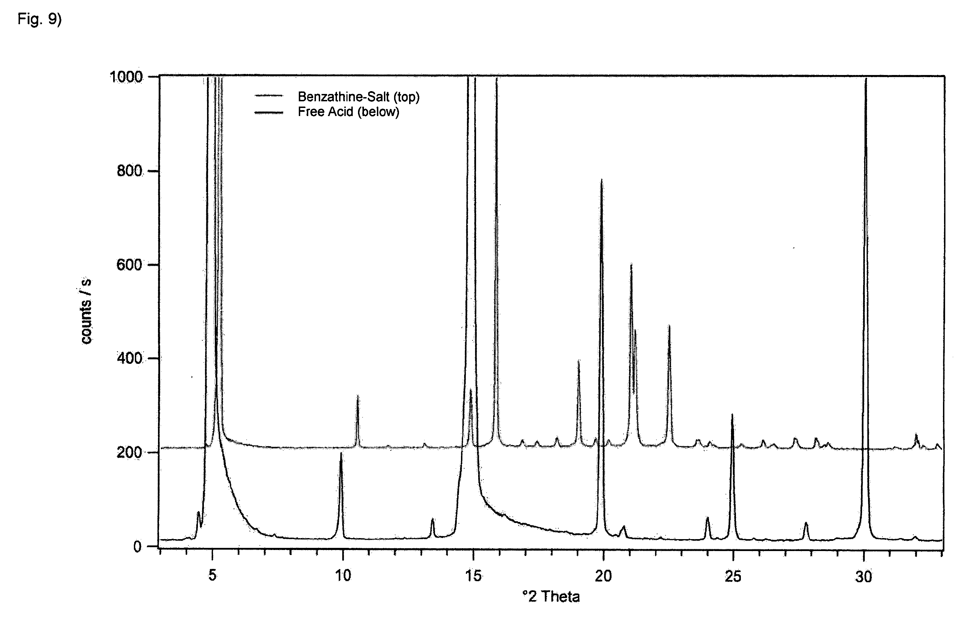

A Bruker D8 Advance with Cu-Kα radiation (Cu_{Kα1} 1.54060A), and LynxEye detector was used. The measurement conditions were 40 kV/40 mA tube power; 0.02· 28 step size, 37 s step time, 2.550· 28 scanning range. The samples were measured under ambient air atmosphere without any special treatment other than the application of slight pressure to get a flat surface; silicon single crystal sample holder, 0.1-mm deep. The PXRD pattern (Fig. 9) of the new salt (top) differs from that of the free phenylbutyric acid (lower) and confirms the crystallinity of the new salt.

The raw data for the results show a PXRD pattern with peaks [28] [°] and [d-Value] [A] at:

**Table 6)**

| **Angle 2-** 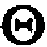 **°** | **d-Value A** | **Intensity %** |
|---|---|---|
| 5.241 | 16.8488 | 100.0 |
| 10.541 | 8.3855 | 2.8 |
| 13.117 | 6.7442 | 0.5 |
| 14.878 | 5.9498 | 3.3 |
| 15.837 | 5.5915 | 25.1 |
| 16.857 | 5.2553 | 0.7 |
| 17.402 | 5.0920 | 0.6 |
| 18.171 | 4.8782 | 0.8 |
| 19.019 | 4.6626 | 5.3 |
| 19.657 | 4.5127 | 0.8 |
| 20.177 | 4.3974 | 0.7 |
| 21.042 | 4.2186 | 10.5 |
| 21.200 | 4.1876 | 7.4 |
| 22.485 | 3.9511 | 7.3 |
| 23.601 | 3.7667 | 0.8 |
| 24.069 | 3.6945 | 0.7 |
| 25.275 | 3.5208 | 0.5 |
| 26.121 | 3.4087 | 0.8 |
| 27.352 | 3.2581 | 0.9 |
| 28.160 | 3.1664 | 0.9 |
| 28.624 | 3.1161 | 0.6 |
| 31.174 | 2.8668 | 0.4 |
| 32.008 | 2.7940 | 1.2 |
| 32.263 | 2.7725 | 0.5 |
| 32.796 | 2.7286 | 0.6 |
| 33.565 | 2.6678 | 0.5 |
| 34.964 | 2.5642 | 0.4 |
| 36.362 | 2.4687 | 0.6 |
| 37.518 | 2.3953 | 2.7 |
| 38.618 | 2.3296 | 0.6 |

### c) ¹H-NMR:

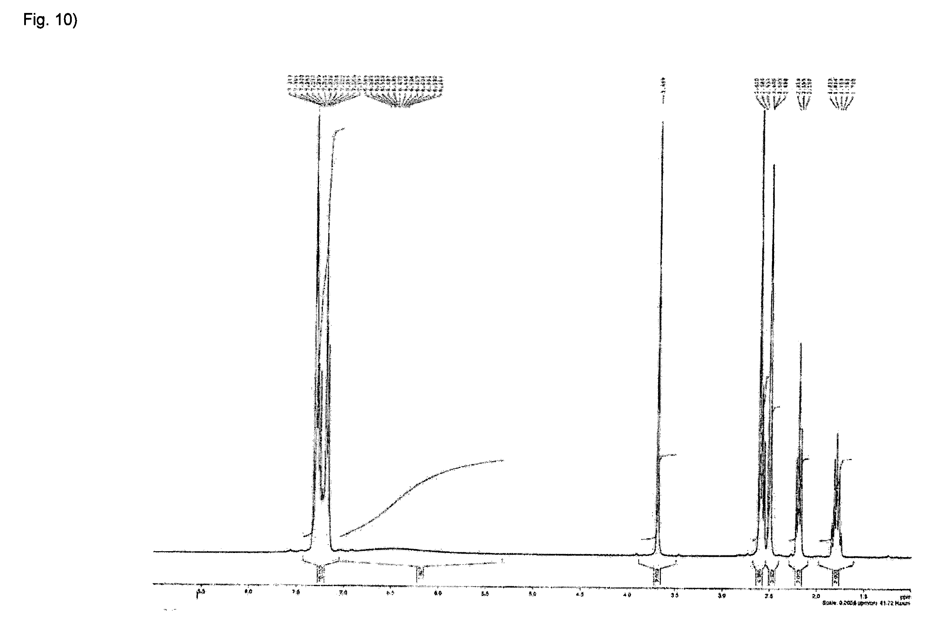

A Bruker DPX300 spectrometer with a proton frequency of 300.13 MHz, 30° excitation pulse, a recycle delay of 1 s at 300.13 MHz. 16 scans were accumulated and d₆-DMSO used as solvent. The ¹H-NMR spectrum (as well as the elemental analysis) agree with a 2 : 1 salt formation/a henibenzathine salt (Figure 10; as well as Table 7 below).

### d) Elemental Analysis:

A Leco CHN 800 was used for C, H, and N determination by combustion and a Leco RO-478 for determination of O by pyrolysis.

**Table 7): Elemental analysis of Example 2**

| | %C | %H | %N | % O | Σ |
|---|---|---|---|---|---|
| Example 2 (experimental determination) | 76.1 | 7.6 | 5.2 | 11.3 | 100.2 |
| Example 2 (experimental determination; normalized) | 76.0 | 7.6 | 5.2 | 11.2 | 100.0 |
| C₁₀H₁₂O₂ • 0.5 C₁₆H₂₀N₂ (theoretical 2 : 1 salt) | 76.0 | 7.8 | 4.9 | 11.3 | 100.0 |
| Difference (experimental (normalized) - theoretical) | 0.0 | -0.2 | 0.3 | -0.1 | -- |

### e) Optical Microscopy:

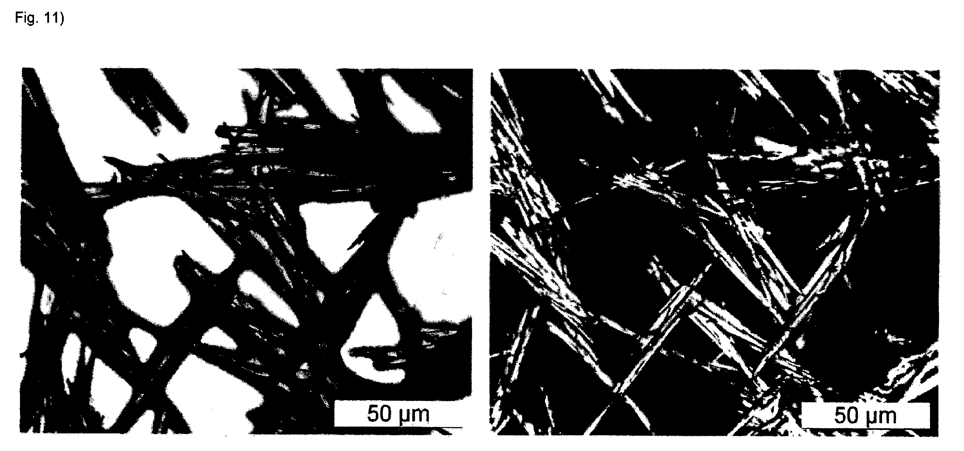

A Leitz Orthoplan 110680 microscope equipped with a Leica DFC280 camera and IM50 v.5 image-capturing software was used. Images were recorded with or without crossed polarizers and with 4x, 10x, or 25x magnification. Fig. 11) shows crystalline rods or thick needles (without crossed polarizers [left]; with crossed polarizers [right]).

### f) TG-FTIR:

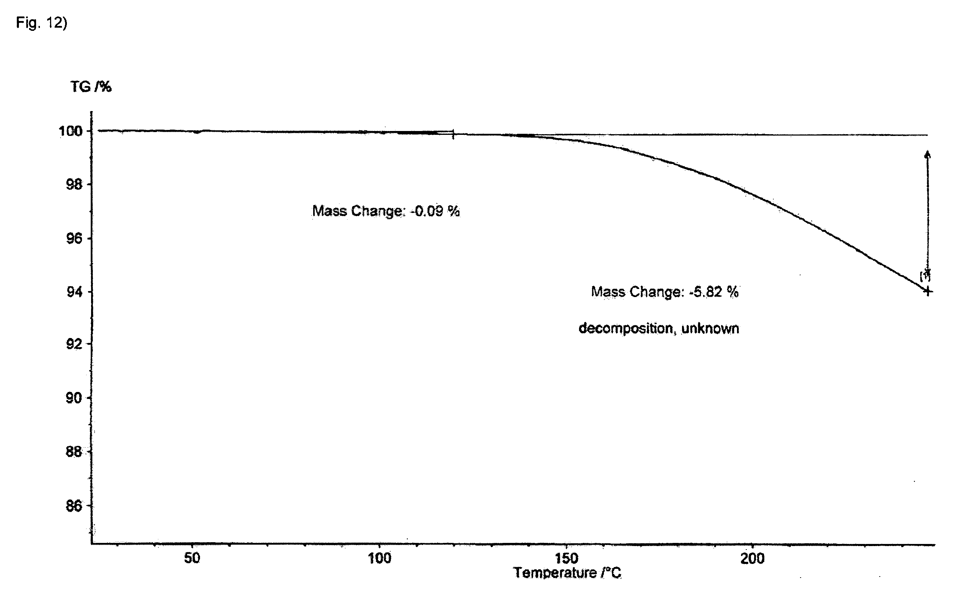

For the TG-FITR thermogram a Netzsch Thermo-Microbalance TG 209 with Bruker FT-IR Spectrometer Vector 22 and a Al crucible (with microhole) was used under N₂ atmosphere at 10 K/min heating rate in 25-250°C range. The TG-FTIR thermogram (Fig. 12) shows a mass loss of ~0.1 wt.-% from 25-120°C which indicates that this salt is anhydrous. Further mass loss is observed at temperatures >120°C.

### g) DSC:

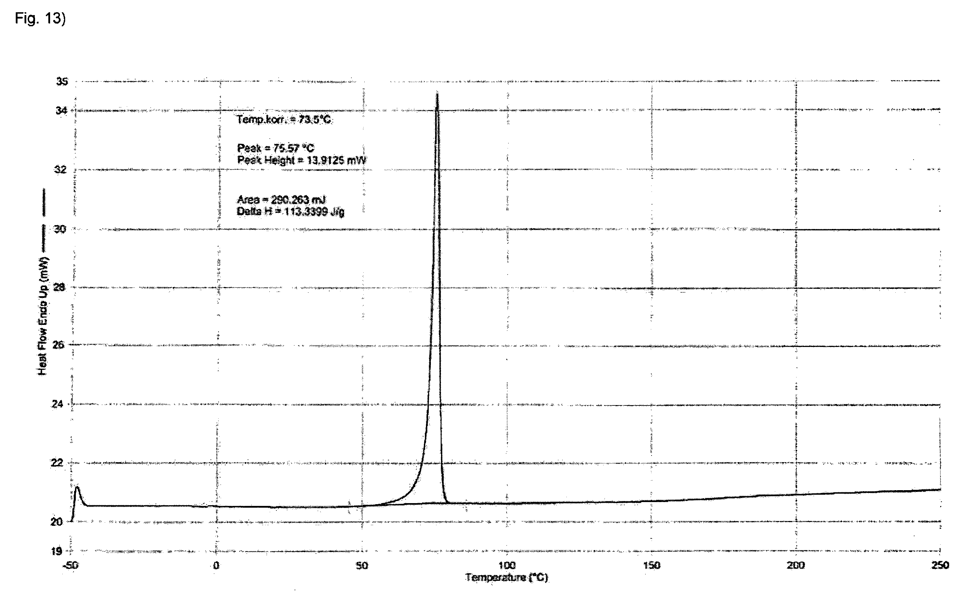

A Perkin Elmer DSC 7 with closed gold crucibles was used. The sample was filled and dried in an N₂ environment. The heating rate was 10 K/min between -50 to 250°C or 350°C range. The DSC thermogram (Figure 13) shows a melting endotherm at 74°C (ΔH = 113.3 J/g) and no further thermal event up to 250°C.

### h) DVS:

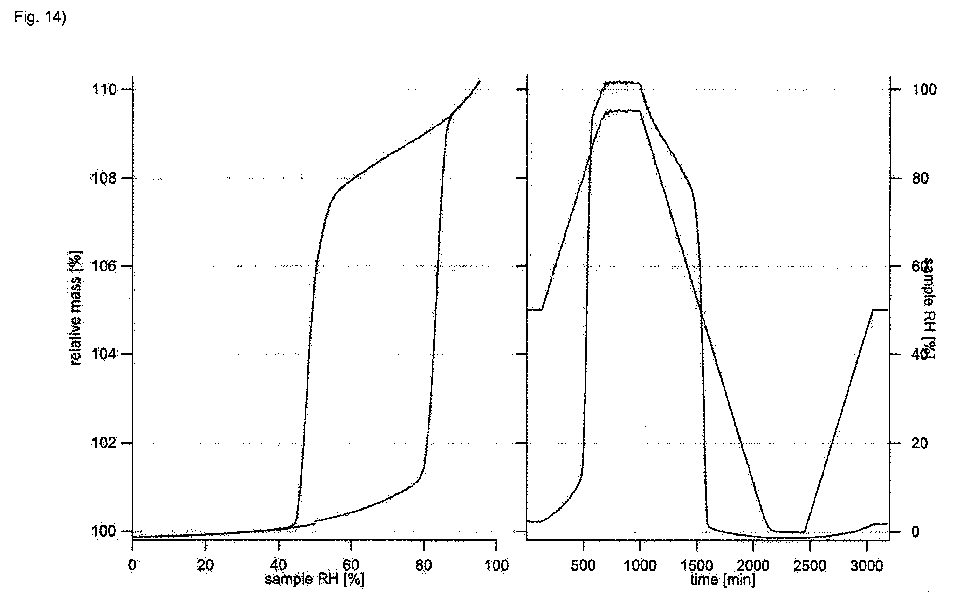

For the measurement of hygroscopy of the salt a Projekt Messtechnik Sorptions Prüfsystem SPS 11 - 100n was used. (Hygroscopy of the phenylbutyric acid for comparison was measured with the Surface Measurement Systems DVS-1). The salt sample was placed on an aluminium (SPS 11) holder (for phenylbutyric acid on a platinum holder (DVS-1)) on top of a microbalance and allowed to equilibrate for 2 h at 50% r.h. before starting the pre-defined humidity program:
(1)2hat50%r.h.
(2) 50 → 95% r.h. (5%/h); 5 h at 95% r.h.
(3) 95 → 0% r.h. (5%/h); 5 h at 0% r.h.
(4) 0 → 50% r.h. (5%/h); 2 h at 50% r.h.
The results are shown in Fig. 14). The DVS isotherm (Figure 14) shows a gradual mass gain of -1.3 wt.-% upon increasing the relative humidity from 50 to 80% r.h. However, raising the relative humidity further from 80 to 95% r.h. results in a steep gain of -10 wt.-%. Upon lowering the relative humidity, the mass decreases only slightly (by -2.2 w1.-%) until a relative humidity of -60 % r.h. is reached. Then a steep loss of -10 wt.-% occurs until the mass of the starting material is reached at -45% r.h. Thus, the mass gain and loss of -10 wt.% is reversible, but shows a large hysteresis. Upon lowering the relative humidity from 50 to 0% r.h. a mass loss of -0.3 wt.-% is observed, but it is fully reversible. At the end of the cycle the sample had essentially the same mass as the starting material. The FT-Raman spectrum of the sample after the DVS measurement shows no changes. A sesquihydrate of the hemi-benzathine salt (1 :0.5:1.5 phenylbutyrate:benzathine:H₂O) has a theoretical water content of 9.5 wt.-%. A reversible hydrate formation occurred during the DVS experiment.

### i) Aqueous Solubility Determination:

For this measurement approximately 1.0 mL of doubly distilled water was added to 18 mg of the substance according Example 1 to be measured. The resulting suspension was equilibrated in a temperature-controlled *Eppendorf Thermomixer Comfort* shaker for 24 h at 25°C at a shaking rate of 600 rpm. The solid phase was recovered by filter centrifugation (0.10-µm PVDF membrane) and examined by FT-Raman spectroscopy. Concentrations in the filtrate (i.e., saturated solutions) were determined by HPLC (see below). The pH of the saturated solution was determined with a Metrohm 713 pH meter.
**HPLC**: *TSP* instrument with P4000 pump, AS3000 autosampler, SCM1000 degasser, UV3000 detector, and PC1000 Vers. 4.1 software.

**Table 8: HPLC method used for solubility determinations.**

| | | | |
|---|---|---|---|
| Column | Waters, XTerra MS C18, 100 mm x 4.6 mm, 5-µm particles (CC01) | | |
| Mobile phase A | H₂0/MeCN 95:5 + 0.1 % TFA | | |
| Mobile phase B | H₂0/MeCN 5:95 + 0.1 % TFA | | |
| Reference conc. | ca. 0.2 mg/mL | | |
| Retention time | 7.7-8.0 min | | |
| Isocratic | 0 min | 75% A | 25% B |
| | 15 min | 75% A | 25% B |
| Flow | 1.0mL/min | | |
| Injection volume | 10 µl | | |
| Wavelength | 207 nm | | |

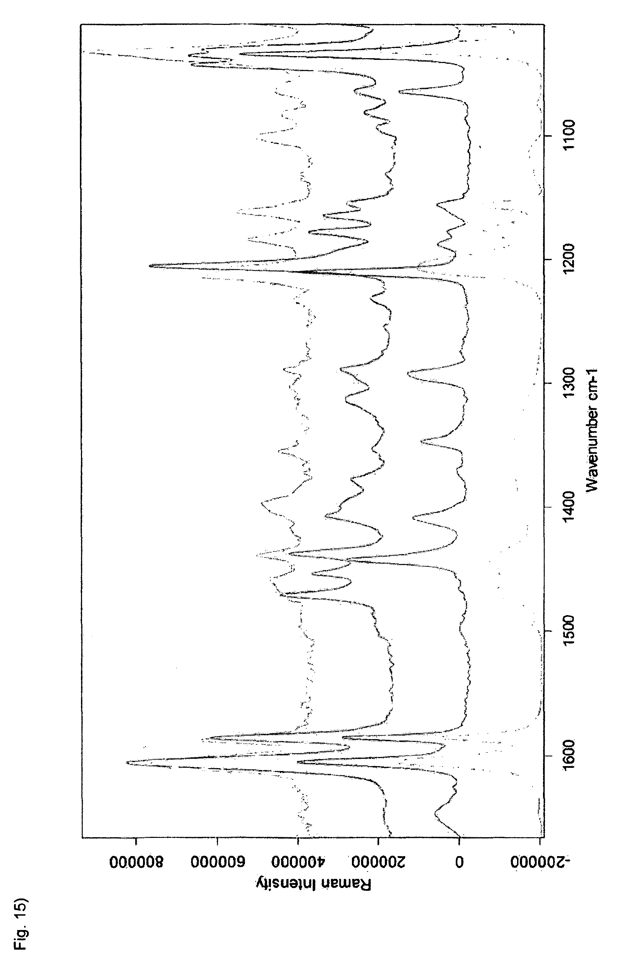

An aqueous solubility (after 24 h of equilibration at 25°C) of 1.7 mg/mL was determined. The pH of the corresponding saturated solution was 6.2. However, the Raman spectrum of the solid residue is unknown (Figure 15; top), it does not correspond to that of the benzathine salt (Figure 15; second from top), the free acid (third from top) or the salt former benzathine (lowest). Considering the information from the DVS measurement, a hydrate is formed and precipitated during equilibration in H₂0. Thus, the solubility value measured corresponds to a hydrate of the benzathine salt and not to the anhydrous form.

### Summary:

The 4-Phenylbutyrate-Hemi-Benzathine salt having a molecular ratio of 2:1 is a crystalline anhydrous salt. The salt has with 74°C a significantly higher melting point than the phenylbutyric acid (51 °C). This salt also opens the path to develop an alternative to the known sodium phenylbutyrate avoiding sodium, while its ratio also allows for a higher proportion of active agent compared to the counter ion.

### Example 3: Small-Scale production of 4-Phenylbutyrate-Benzylamine salt and characterization

### Production:

a) A solution of 0.05 mol/l of 4-Phenylbutyric acid in ethanol was prepared (Stock A1).
   A second solution of 0.05 mol/l benzylamine in ethanol was prepared (Stock B1). 100 µl of the 4-Phenylbutyric acid solution (Stock A1) was mixed with 100 µl of the benzylamine solution (Stock B1). The solvent was removed under N₂ flow at room temperature and a crystalline solid was obtained.
b) A solution of 0.05 mol/l of 4-Phenylbutyric acid in THF was prepared (Stock A2). A second solution of 0.05 mol/l benzylamine in THF was prepared (Stock B2). 100 µl of the 4-Phenylbutyric acid solution (Stock A2) was mixed with 100 µl of the benzylamine solution (Stock B2). The solvent was removed under N₂ flow at room temperature and a crystalline solid was obtained.
c) A solution of 0.05 mol/l of 4-Phenylbutyric acid in MeCN was prepared (Stock A3). A second solution of 0.05 mol/l benzylamine in MeCN was prepared (Stock B3). 100 µl of the 4-Phenylbutyric acid solution (Stock A3) was mixed with 100 µl of the benzylamine solution (Stock B3). The solvent was removed under N₂ flow at room temperature and a crystalline solid was obtained.

### Characterization:

### FT-Raman Spectroscopy:

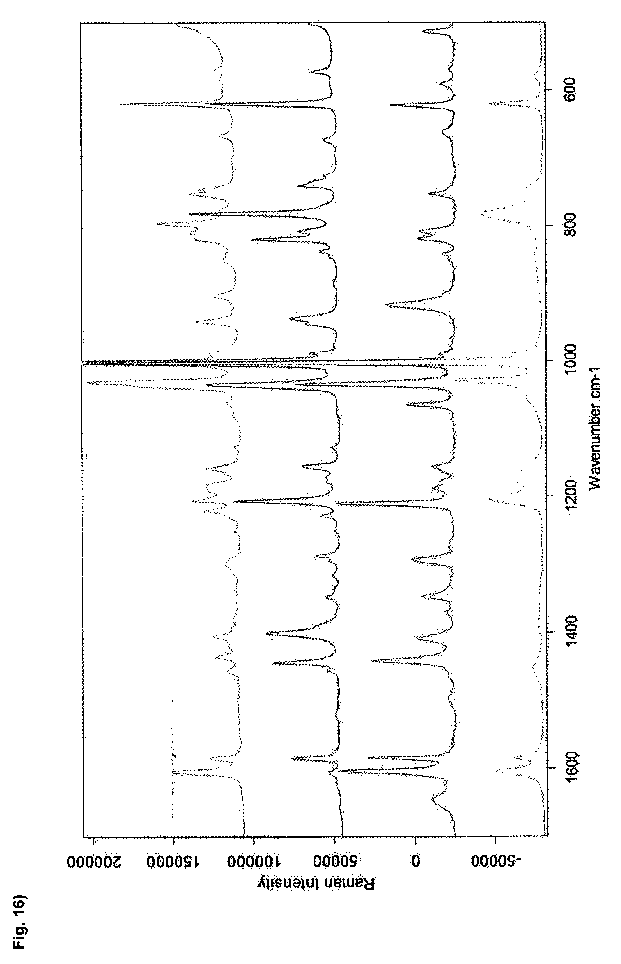

The crystalline residue was examined by FT-Raman Spectroscopy. A Bruker RFS100 with OPUS 6.5 software was used having an Nd:YAG 1064-nm excitation and a Ge detector, 3500-100 cm⁻¹ range. The measurement conditions were 100-300 mW laser power, 64 - 128 scans, 2 cm⁻¹ resolution. The Raman spectrum (Fig. 16) shows the spectrum of a crystalline form (top) of a phenylbutyrate-benzylamine salt according to Example 3) as well as a spectrum of an amorphous form of a phenylbutyrate-benzylamine salt according to Example 4) (2^{nd} from top of Fig. 16). Fig. 16) shows that both the new spectra are different from the spectrum of either the free phenylbutyric acid (third from top) or the benzylamine salt-former (lowest).

The raw data for the results of the crystalline form of a phenylbutyrate-benzylamine salt according to Example 3) show an FT-Raman pattern with peaks [cm⁻¹] at:

**Table 9)**

| **peaks [cm**⁻¹**]** | **abs. int.** |
|---|---|
| 1606 | 48359 |
| 1585 | 25812 |
| 1437 | 22323 |
| 1406 | 23170 |
| 1300 | 16908 |
| 1221 | 27749 |
| 1206 | 35023 |
| 1185 | 26991 |
| 1159 | 27540 |
| 1031 | 92016 |
| 1002 | 322442 |
| 941 | 32264 |
| 904 | 23165 |
| 811 | 35205 |
| 797 | 53797 |
| 753 | 36152 |
| 746 | 30463 |
| 667 | 18740 |
| 621 | 73748 |
| 571 | 21526 |
| 487 | 81165 |
| 392 | 62464 |
| 270 | 58477 |
| 219 | 70471 |
| 196 | 82197 |

### Example 4: Small-Scale production of 4-Phenylbutyrate-Benzylamine amorphous form and characterization

### Production:

a) A solution of 0.05 mol/I of 4-Phenylbutyric acid in acetone was prepared (Stock A4).
   A second solution of 0.05 mol/l benzylamine in acetone was prepared (Stock B4). 100 µl of the 4-Phenylbutyric acid solution (Stock A4) was mixed with 100 µl of the benzylamine solution (Stock B4). The solvent was removed under N₂ flow at room temperature and a partly crystalline amorphous form was obtained.
b) To the crystalline solid of example 3 b (from EtOH solvent) 100 µl of TBME was added. The mixture was stirred for 4 days with the following temperature cycling program: holding at 20°C for 1 h, heating to 30°C in 1 h, holding at 30°C for 1 h, cooling to 20°C in 1 h. A partly crystalline amorphous form was obtained.

### Characterization:

### FT-Raman Spectroscopy:

The residue was examined by FT-Raman Spectroscopy. A Bruker RFS100 with OPUS 6.5 software was used having an Nd:YAG 1064-nm excitation and a Ge detector, 3500-100 cm⁻¹ range. The measurement conditions were 100-300 mW laser power, 64 - 128 scans, 2 cm⁻¹ resolution. The Raman spectrum (Fig. 16) shows the spectrum of a crystalline form (top) of a phenylbutyrate-benzylamine salt according to Example 3) as well as a spectrum of an amorphous form of a phenylbutyrate-benzylamine salt according to Example 4) (2^{nd} from top of Fig. 16). Fig. 16) shows that both the new spectra are different from the spectrum of either the free phenylbutyric acid (third from top) or the benzylamine salt-former (lowest).

### Example 5: Small-Scale production of 4-Phenylbutyric acid-gentisic acid co-crystal and characterization

### Production:

a) A solution of 0.05 mol/I of 4-Phenylbutyric acid in acetone was prepared (Stock A4).
   A second solution of 0.05 mol/I gentisic acid in acetone was prepared (Stock C4). 100 µl of the 4-Phenylbutyric acid solution (Stock A4) was mixed with 100 µl of the gentisic acid solution (Stock C4). The solvent was removed under N₂ flow at room temperature and a crystalline form was obtained.
   To the crystalline form 100 µl of toluene was added. The mixture was stirred for 4 days with the following temperature cycling program: holding at 20°C for 1 h, heating to 30°C in 1 h, holding at 30°C for 1 h, cooling to 20°C in 1 h. A crystalline form was obtained
b) A solution of 0.05 mol/l of 4-Phenylbutyric acid in EtOH was prepared (Stock A1). A second solution of 0.05 mol/I gentisic acid in EtOH was prepared (Stock C1). 100 µl of the 4-Phenylbutyric acid solution (Stock A1) was mixed with 100 µl of the gentisic acid solution (Stock C1). The solvent was removed under N₂ flow at room temperature and a crystalline form was obtained.
   To the crystalline form 100 µl of TBME was added. The mixture was stirred for 4 days with the following temperature cycling program: holding at 20°C for 1 h, heating to 30°C in 1 h, holding at 30°C for 1 h, cooling to 20°C in 1 h. A crystalline form was obtained.

### Characterization:

### FT-Raman Spectroscopy:

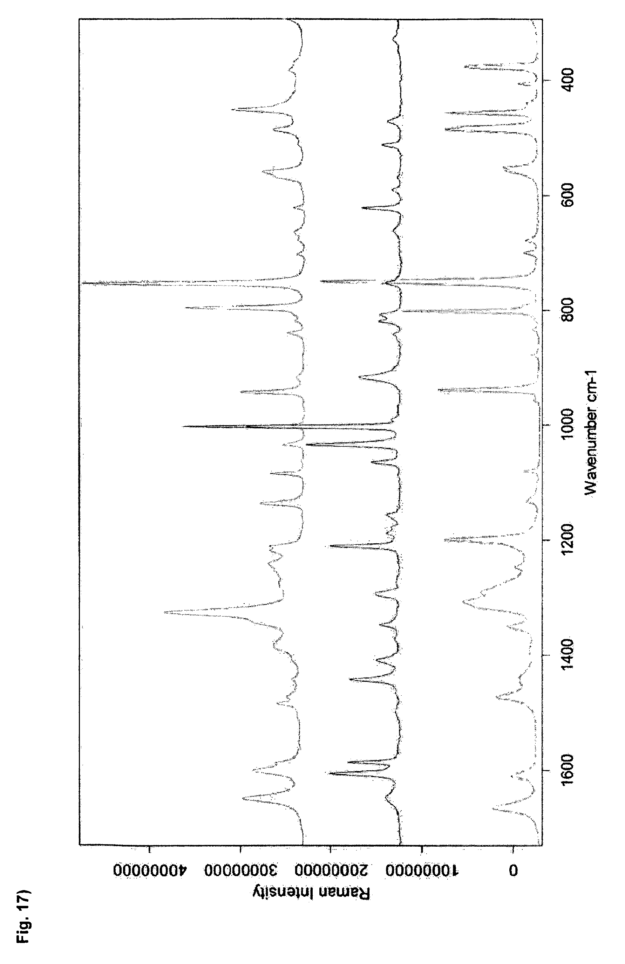

The residue was examined by FT-Raman Spectroscopy. A Bruker RFS100 with OPUS 6.5 software was used having an Nd:YAG 1064-nm excitation and a Ge detector, 3500-100 cm⁻¹ range. The measurement conditions were 100-300 mW laser power, 64 - 128 scans, 2 cm⁻¹ resolution. The Raman spectrum (Fig. 17) shows the spectrum of the co-crystal (top) of phenylbutyric acid with gentisic acid and demonstrates that the spectrum of the new co-crystal is different from the spectrum of either the free phenylbutyric acid (middle) or the gentisic acid co-crystal-former (lowest).

The raw data for the results of the co-crystal of phenylbutyric acid with gentisic acid according to Example 5) show an FT-Raman pattern with peaks [cm⁻¹] at:

**Table 10)**

| **peaks [cm**⁻¹**]** | **abs. int.** |
|---|---|
| 1648 | 34800 |
| 1600 | 31410 |
| 1484 | 23251 |
| 1384 | 25723 |
| 1326 | 69407 |
| 1242 | 29205 |
| 1211 | 29177 |
| 1136 | 33859 |
| 1085 | 30379 |
| 1035 | 25891 |
| 1003 | 41098 |
| 943 | 44030 |
| 840 | 26604 |
| 796 | 68084 |
| 754 | 109329 |
| 703 | 24513 |
| 665 | 26258 |
| 622 | 26839 |
| 560 | 40087 |
| 487 | 37208 |
| 452 | 54231 |
| 382 | 32884 |
| 288 | 41352 |
| 187 | 111172 |

### Example 6: Slow Release Tablets with 4-Phenylbutyrate-Mono-Benethamine salt produced e.g. according to Example 1

A mixture of 6,280.0 g of lactosum monohydricum, 3,500.0 g of Methocel K100 MPremium (Prachem), and 750.0 g of Avicel PH 102 (Select Chemie) together with 4-phenylbutyrate-mono-benethamine salt in various amounts ranging from 10 to 40 or 15 to 35 moles of the salt (e.g. 12,000.0 g [or 12,100.0 g] or 6,000.0 g [or 6,050.0 g]), is wetted with 4,000.0 g of aqua purificata (water purified by inversion osmosis) and dried in cold air during 18 hours. The mixture is forced through a sieve IV mm and dried again during 10 hours with air of 40°C. A mixture of 240.0 g of talcum and 30.0 g of magnesium stearate is admixed during 20 minutes and the mixture is pressed into tablets of 0.70 g each, a thickness of about 6.8 mm and with a hardness of 90 Newton. Yield: 24,000 tablet cores.

The mixing is carried out with a Diosna Mixer, the drying in a Lükon drying cabinet, the sieving with a Köhler & Bosshard sieving machine, and the tablet pressing with a Korsch tablet press EK 11. The cores are provided with a film coating for example by using a colloidal dispersion containing 7,850 g of isopropylalcohol, 3,360 g of Eudragit L 12.5, 66 g of dibutyl phthalat, 18.0 g of Miglyol 812, and 56 g of polyethylenglycol PEG 400. The suspension is sprayed at 3.5 bar and 25° C onto the 24,000 cores. The film-coated tablets are dried in a circulating air drying cabinet for at least 4 hours at 35°C.

### Example 7: Slow Release Tablets with 4-Phenylbutyrate-Hemi-Benzathine salt produced e.g. according to Example 2

A mixture of 6,280.0 g of lactosum monohydricum, 3,500.0 g of Methocel K100 MPremium (Prachem), and 750.0 g of Avicel PH 102 (Select Chemie) together with 4-phenylbutyrate-hemi-benzathine salt in various amounts ranging from 5 to 20 or 7 to 18 moles of the salt (e.g. 6,500.0 g or 6,518.0 g), is wetted with 4,000.0 g of aqua purificata (water purified by inversion osmosis) and dried in cold air during 18 hours. The mixture is forced through a sieve IV mm and dried again during 10 hours with air of 40°C. A mixture of 240.0 g of talcum and 30.0 g of magnesium stearate is admixed during 20 minutes and the mixture is pressed into tablets of 0.70 g each, a thickness of about 6.8 mm and with a hardness of 90 Newton. Yield: 24,000 tablet cores.

The mixing is carried out with a Diosna Mixer, the drying in a Lükon drying cabinet, the sieving with a Köhler & Bosshard sieving machine, and the tablet pressing with a Korsch tablet press EK 11. The cores are provided with a film coating for example by using a colloidal dispersion containing 7,850 g of isopropylalcohol, 3,360 g of Eudragit L 12.5, 66 g of dibutyl phthalat, 18.0 g of Miglyol 812, and 56 g of polyethylenglycol PEG 400. The suspension is sprayed at 3.5 bar and 25° C onto the 24,000 cores. The film-coated tablets are dried in a circulating air drying cabinet for at least 4 hours at 35°C.

## Claims

1. Joined mixtures of phenylbutyric acid or its inorganic salts and at least one organic molecule, **characterized in that** the mixture is joined by intermolecular physically interacting forces,
with the proviso that salts of phenylbutyric acid with ammonium, 1-carboxy-N,N,N-trimethyl-methanaminium
or co-crystals with sodium phenylbutyrate are excluded.

2. Joined mixture according to claim 1, **characterized in that** the joined mixture is selected from
• a salt of phenylbutyrate with a positively charged ion of an organic molecule including a respective polymorph, hydrate or solvate of the salt;
• a salt of phenylbutyrate with a positively charged ion of an organic molecule;
• a polymorph of a salt of phenylbutyrate with a positively charged ion of an organic molecule;
• a hydrate of a salt of phenylbutyrate with a positively charged ion of an organic molecule;
• a solvate of a salt of phenylbutyrate with a positively charged ion of an organic molecule;
• a co-crystal of phenylbutyric acid and at least one organic molecule as co-crystal former including a respective polymorph, hydrate or solvate of the co-crystal;
• a co-crystal of phenylbutyric acid and at least one organic molecule as co-crystal former;
• a polymorph of a co-crystal of phenylbutyric acid and at least one organic molecule as co-crystal former;
• a hydrate of a co-crystal of phenylbutyric acid and at least one organic molecule as co-crystal former; or
• a solvate of a co-crystal of phenylbutyric acid and at least one organic molecule as co-crystal former.

3. Joined mixture according to any of claims 1 or 2, **characterized in that** the organic molecule is selected from
• ammonia and amines,
• amino acids,
• carboxylic acids, or
• heterocylic compounds
preferably the organic molecule is selected from
• ammonia;
• amines, like 2-(diethylamino)-ethanol, benethamine, benzathine, benzylamine, betaine (trimethylglycine), choline, deanol, diethanolamine, diethylamine (2,2'-iminobis(ethanol)), ethanolamine (2-aminoethanol), ethylenediamine, glucamines, like N-methyl-glucamine, hydrabramine, tromethamine, triethanolamine (2,2',2''-nitrolitris(ethanol)):
• carbocylic acids, like benzoic acid, citric acid, gentisic acid, glycolic acid, lactic acid, naphtoic acids, succinic acid, tartaric acid, or vanillic acid;
• D-aminoacids like D-alanine:
• L-amino acids like L-lysine or L-arginine; or
• heterocyclic compounds, like imidazoles, like 1 *H*-Imidazole; morpholines, like 4-(2-hydroxyethyl)-morpholine; piperazine; pyrrolidines, like 1-(2-hydroxyethyl)-pyrrolidine.

4. Joined mixture according to any of claims 1 to 3, **characterized in that** the organic molecule is selected from
• 2-(diethylamino)-ethanol,
• ammonia,
• benethamine,
• benzathine,
• benzoic acid,
• benzylamine,
• betaine (trimethylglycine),
• choline,
• citric acid,
• D-alanine,
• deanol,
• diethanolamine,
• diethylamine (2,2'-iminobis(ethanol)),
• ethanolamine (2-aminoethanol),
• ethylenediamine,
• gentisic acid,
• glucamines, like N-methyl-glucamine,
• glycolic acid,
• hydrabramine,
• imidazoles, like 1 *H*-Imidazole,
• lactic acid,
• L-amino acids like L-lysine or L-arginine,
• morpholines, like 4-(2-hydroxyethyl)-morpholine,
• naphtoic acids,
• piperazine,
• pyrrolidines, like 1-(2-hydroxyethyl)-pyrrolidine,
• succinic acid,
• tartaric acid,
• tromethamine,
• triethanolamine (2,2',2''-nitrolitris(ethanol)), or
• vanillic acid;
preferably is selected from
• 1-(2-hydroxyethyl)-pyrrolidine,
• 2-(diethylamino)-ethanol,
• 4-(2-hydroxyethyl)-morpholine,
• benethamine,
• benzathine,
• benzoic acid,
• benzylamine,
• betaine (trimethylglycine),
• choline,
• citric acid,
• deanol,
• diethylamine,
• ethanolamine,
• gentisic acid,
• glycolic acid,
• hydrabramine,
• lactic acid,
• L-arginine,
• L-lysine,
• naphtoic acids,
• N-methyl-glucamine,
• piperazine
• succinic acid,
• tartaric acid,
• tromethamine, or
• vanillic acid;
more preferably is selected from
• 1-(2-hydroxyethyl)-pyrrolidine, or
• 2-(Diethylamino)-ethanol,
• 4-(2-hydroxyethyl)-morpholine,
• benethamine,
• benzathine,
• benzoic acid,
• benzylamine,
• betaine (trimethylglycine),
• choline,
• citric acid,
• D-alanine,
• deanol,
• diethylamine,
• gentisic acid,
• glycolic acid,
• hydrabramine,
• lactic acid,
• L-arginine,
• L-lysine,
• naphtoic acids,
• N-methyl-glucamine,
• succinic acid,
• tartaric acid,
• tromethamine, or
• vanillic acid;
most preferably is selected from
• 1-(2-hydroxyethyl)-pyrrolidine, or
• 2-(diethylamino)-ethanol,
• 4-(2-hydroxyethyl)-morpholine,
• benethamine,
• benzathine,
• benzoic acid,
• benzylamine,
• citric acid,
• deanol,
• diethylamine,
• gentisic acid,
• glycolic acid,
• hydrabramine,
• lactic acid,
• naphtoic acids,
• N-methyl-glucamine,
• succinic acid,
• tartaric acid,
• tromethamine, or
• vanillic acid;
• or - optionally - D-alanine.

5. Joint mixture according to any of claims 1 to 4, wherein the joint mixture is in crystalline form.

6. Joint mixture according to any of claims 1 to 4, being a polymorph of a crystalline form according to claim 5 or being an amorphous form.

7. Pharmaceutical formulation comprising at least one joint mixture according to any of claims 1 to 6 and optionally at least one physiologically acceptable excipient.

8. Joint mixture according to any of claims 1 to 6 for use in the treatment of cancer, mucositis, amyotropic lateral sclerosis (ALS), skin/mucosal itching, degenerative diseases of the eye, neurodegenerative disorders, Huntington's disease, eating disorders, obesity, drug induced weight gain, pruritis, alcoholism, promyelocytic leukemia, especially acute promyelocytic leukemia, cardiac injury, especially adriamycin induced cardiac injury, epithelial damage, connective tissue damage, desmosis, Parkinson Disease, myelodysplastic syndrome, urea cycle disorder, fibrotic lung diseases, hepatic encephalopathies, thioredoxin mediated diseases, human papilloma virus (HPV) infections, autoimmune diseases, thalassemia, genetic disorders; or in the regulation of HDAC6 activity, the modulation of stem cells, the prevention of grey hair, the promotion of neuronal growth, the prevention of bone and joint diseases, or as adjuvant in cancer therapy.

9. Process for the production of a joint mixture according to any of claims 1 to 6 with the following steps:
a) 4-phenyl-butyric acid is dissolved in organic solvent 1 to form solution 1;
b) the organic molecule is dissolved in organic solvent 2 to form solution 2;
c) solution 1 and solution 2 are mixed;
d) in a first final step the organic solvents are removed from the mixture from step c) and a residue/joined mixture obtained.

10. Joint mixture according to any of claims 1 to 6, wherein the joint mixture is a salt of 4-phenyl-butyric acid with a salt-forming organic molecule.

11. A salt according to claim 10, being a salt of 4-phenyl-butyric acid with a salt-forming organic molecule selected from
• benethamine,
• benzathine,
• benzylamine,
• N-methyl-glucamine,
• 4-(2-hydroxyethyl)-morpholine, or
• 1-(2-hydroxyethyl)-pyrrolidine;
or being a salt of 4-phenyl-butyric acid with a salt-forming organic molecule according to general formula II 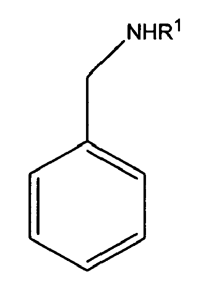 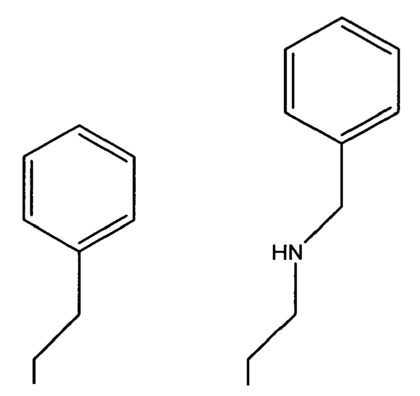 wherein R¹ is selected from H,

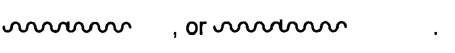

12. The salt according to claim 11, wherein the salt-forming organic molecule according to general formula II is benzylamine.

13. The salt according to claim 11, wherein the salt-forming organic molecule according to general formula II is benethamine, preferably where the molecular ratio between 4-phenyl-butyric acid and benethamine is between 4:1 and 1:4, preferably is between 2:1 and 1:2, preferably is 1:1.

14. The salt according to claim 11, wherein the salt-forming organic molecule according to general formula II is benzathine, preferably where the molecular ratio between 4-phenyl-butyric acid and benzathine is between 4:1 and 1:4, preferably is between 3:1 and 1:1, or most preferably is 2:1.

15. Joint mixture according to any of claims 1 to 6, wherein the joint mixture is a co-crystal of 4-phenyl-butyric acid or salt thereof with a co-crystal-forming organic molecule;
preferably being an organic acid, preferably is a carboxylic acid, more preferably is an organic acid selected from
• benzoic acid,
• citric acid,
• gentisic acid,
• glycolic acid,
• lactic acid, preferably L-actic acid,
• naphtoic acid, 1-hydroxy-2-,
• succinic acid,
• tartaric acid,
• vanillic acid, preferably L-tartaric acid, or
• a D-amino acid, like D-alanine;
most preferably being gentisic acid.
